# EUROPEAN PATENT SPECIFICATION

(11) **EP 1 465 585 B1**
(45) Date of publication and mention of the grant of the patent: **29.04.2009**
(21) Application number: 02791852.3
(22) Date of filing: 19.12.2002
(51) Int. Cl.: A61K 8/26, A61K 8/34, A61K 8/49, A61Q 15/00, C07D 241/08

(54) **COSMETIC COMPOSITIONS COMPRISING A CYCLODIPEPTIDE COMPOUND**
KOSMETISCHE ZUSAMMENSETZUNGEN ENTHALTEND EIN CYCLODIPEPTID
COMPOSITIONS COSMETIQUES CONTENANT UN COMPOSE CYCLODIPEPTIDIQUE

(30) Priority: 18.01.2002 GB 0201164; 01.08.2002 GB 0217840
(43) Date of publication of application: 13.10.2004
(73) Proprietor: Unilever PLC, London EC4Y 0DY (GB); Unilever N.V., 3013 AL Rotterdam (NL)
(72) Inventor: FRANKLIN, Kevin, R. Unilever R & D Port Sunlight, Bebington Wirral, Merseyside CH63 3JW (GB)
(74) Representative: Pearce, Timothy
(86) International application number: PCT/EP2002/014523
(87) International publication number: WO 2003/059306

(56) References cited:
- JP-A- 07 247 475
- JP-A- 10 226 615
- JP-A- 2001 247 451
- HANBUSA K ET AL: "Cyclo(dipeptide)s as Low-molecular-mass Gelling Agents to Harden Organic Fluids" JOURNAL OF THE CHEMICAL SOCIETY, CHEMICAL COMMUNICATIONS, CHEMICAL SOCIETY. LETCHWORTH, GB, 1994, pages 1401-1402, XP002191895 ISSN: 0022-4936 cited in the application
- K. HANABUSA, M. MATSUMOTO ET AL.: "Low Molecular Weight Gelators for Organic Fluids: Gelation using a family of Cyclo(dipeptides)" JOURNAL OF COLLOID AND INTERFACE SCIENCE, vol. 224, 2000, pages 231-244, XP002237792 cited in the application

## Description

### FIELD OF THE INVENTION

The present invention relates to cosmetic compositions for application to human skin, to the preparation and use of such compositions and to structurants for incorporation in such compositions and their preparation.

### BACKGROUND OF THE INVENTION AND SUMMARY OF PRIOR ART

A wide variety of cosmetic compositions for application to human skin make use of a structured liquid carrier to deliver colour or some other active material to the surface of the skin. Significant examples of such cosmetic compositions include antiperspirant or deodorant compositions which are widely used in order to enable their users to avoid or minimise wet patches on their skin, especially in axillary regions or to control or prevent the emission of malodours, which could otherwise arise when the user perspires. Other examples of cosmetic compositions include lip sticks.

Although structuring is a term that has often been employed in respect of materials which structure a carrier liquid, various other terms have been employed alternatively, including thickening, solidifying and gelling.

Antiperspirant or deodorant formulations have been provided with a range of different product forms. One of these is a so-called "stick" which is usually a bar of an apparently firm solid material held within a dispensing container and which retains its structural integrity and shape whilst being applied. In that respect they are representative of cosmetic compositions in stick form containing other active constituents. When a portion of the stick is drawn across the skin surface, a film of the stick composition is transferred to the skin surface. Although the stick has the appearance of a solid article capable of retaining its own shape for a period of time, the material often has a structured liquid phase so that a film of the composition is readily transferred from the stick to another surface upon contact.

Antiperspirant sticks can be divided into three categories. Suspension sticks contain a particulate antiperspirant active material suspended in a structured carrier liquid phase which often is anhydrous and/or in many instances may be water-immiscible. Emulsion sticks normally have a hydrophilic phase, commonly containing the antiperspirant active in solution, this phase forming an emulsion with a second, more hydrophobic, liquid phase. The continuous phase of the emulsion is structured. Solution sticks typically have the antiperspirant active dissolved in a structured liquid phase which is polar and may comprise a polar organic solvent, which is often water-miscible, and the polar phase can contain water.

There is substantial literature on structuring of cosmetic compositions, for example as represented by antiperspirant or deodorant compositions.

Conventionally, many sticks have been structured using naturally-occurring or synthetic waxy materials, in which term we include materials which resemble beeswax, in that they soften progressively with increase in temperature until they are fluid, generally by about 95°C. Examples of wax-structured sticks are described in an article in Cosmetics and Toiletries, 1990, Vol 105, P75-78, in US patents 5169626 and 4725432 and in many other publications, in some of which such materials are called solidifying agents.

More specifically, it has been common practice for sticks to be structured or solidified by incorporating fatty alcohol into the composition, often accompanied by a smaller amount of castor wax. Sticks which are structured with fatty alcohol tend to leave visible white deposits on application to human skin; moreover the deposits can also transfer onto clothing when it comes into contact with the skin and the wearer can, for example, find white marks at the armhole of the sleeveless garment. Fatty alcohols are often regarded as coming within the general category of waxy materials, but we have observed that they are a more significant source of white deposits than various other waxy materials.

Some alternative structurants or solidifying agents to waxy materials have been proposed. For example, the use of dibenzylidene sorbitol (DBS) or derivatives thereof as gellant for a polar or hydrophylic carrier liquid has been proposed in a number of publications such as EP-A-512770, WO-92/19222, US 4954333, US 4822602 and US 4725430. Cosmetic formulations containing such gellants can suffer from a number of disadvantages, including instability in the presence of acidic antiperspirants, and comparatively high processing temperatures needed in the production of sticks.

Other alternative proposed structurants include various classes of esters or amides that are solid at ambient temperature and are capable of solidifying a hydrophobic or water-immiscible liquid carrier. One such class comprises ester or amide derivatives of 12-hydroxystearic acid, as described in inter alia US-A-5750096. Another class of such esters or amides comprises N-acyl amino acid amides and esters, of which N-Lauroyl-L-glutamic acid di-n-butylamide is commercially available from Ajinomoto under their designation GP-1. They are described in US patent 3969087. A further class which has been disclosed as gelling agents comprises the amide derivatives of di and tribasic carboxylic acids set forth in WO 98/27954 notably alkyl N,N'-dialkyl succinamides. Yet other amide structurants for water-immiscible liquid carriers are described in EP-A-1305604.

One further class of compounds that have been contemplated as a gelator for cosmetic oils comprises cyclodipeptides. Such compounds contain a -CO-NH- group, and can be considered to be cyclic derivatives of aminoacids.

Various cyclodipeptides has been described in an article by K Hanabusa et al entitled Cyclo(dipeptide)s as low molecular-mass Gelling Agents to harden Organic Fluids, J. Chem Soc. Commun., 1994 pp1401/2. Various other cyclo(dipeptides) satisfying formula 1 above were described in a second article by Hanabusa et al entitled Low Molecular weight Gelators for Organic Fluids: Gelation using a Family of Cyclo(dipeptide)s, in the Journal of Colloid and Interface Science 224, 231-244 (2000). Further cyclodipeptides as well as cosmetic compositions containing them have been described in Japanese Kokai 10-226615 (1998) or 13-247451 (2001) to Polar Chemical Industries Inc. in which Hanabusa was a named inventor.

In the course of the research leading to the present invention, cyclo dipeptides such as various of those disclosed by Hanabusa were investigated and a sub-class of cyclo dipeptides exhibiting superior gelating properties was identified and described in an as yet unpublished copending application no GB0201164.1 filed in GB on 1B/01/2002.

Without being bound to any specific theory or explanation, we believe that, upon structuring of a water-immiscible oil, a network of fibres is formed of the cyclodipeptides that extends throughout the liquid phase, at least increasing the viscosity of the phase and preferably gelling that phase. Upon heating the gel to the gel melting temperature, the strands of structurant dissolve and the liquid phase becomes more mobile. Within the class of compounds identified as cyclodipeptides, the capability of individual members of that class to form a network and the conditions in which a network forms will vary, as will the stability of a network, once formed. However, the class shares the properties outlined below to a greater or lesser extent.

Although cyclo dipeptides can be extremely effective gellants for cosmetic oils, the manufacture of compositions in which they are employed as gellants is subject to a number of practical constraints or difficulties. First, it is often difficult to incorporate sufficient cyclic dipeptide into the cosmetic oil to enable it to structure or gel the oil to the extent that would be preferred by the manufacturer. That is because the cyclic dipeptides are relatively poorly soluble in commonly employed cosmetic oils, such as volatile or even non-volatile silicone oils. It would be inherently desirable to find a way of increasing the solubility of cyclo dipeptides in the water-immiscible liquid phase of cosmetic formulations.

A further property of cyclic dipeptides relates to the gelling temperature of cosmetic oils containing them. As a generalisation, they tend to gel at higher temperatures than for example waxes or like commonly employed gellants. Furthermore, and unsurprisingly, the gelling temperature of a solution of such a gellant in such oils increases as its concentration in solution increases. The net consequence of its gelation behaviour is that if enough cyclic dipeptide is present to cause the resultant product to have a preferred firmness at ambient temperature, the gelation temperature of the cyclic dipeptide in the oil is undesirably high, commonly in the region of or in excess of 100°C. At such temperatures, many cosmetic oils can evaporate or become discoloured and if the formulation is in the form of an emulsion stick, water evaporation renders the preparation of accurate composition extremely difficult and at worst impossible. Moreover, increased processing temperatures can also result in degradation or discoloration of some cyclic dipeptides themselves. It would be inherently desirable to find a way of lowering the gelling temperature at which a water-immiscible oil phase occurs to a controllable extent.

The instant inventors accordingly concluded that it would be desirable to identify variations in processing that could ameliorate or overcome the foregoing operational constraints, preferably both at the same time.

### SUMMARY OF THE INVENTION

Applicants have now found that the processing of cosmetic formulations employing a cyclodipeptide as a gelling agent for a cosmetic oil carrier can be improved by employing a class of materials that can act as a solvent for the cyclo dipeptides and which is miscible with the cosmetic oils.

It is an object of the present invention to provide structured cosmetic-compositions, in which a liquid carrier material is structured using a cyclo dipeptide structurant in the presence of a solvent for the cycle dipeptide which is miscible with the carrier.

Broadly, in a first aspect of the present invention, there is provided a cosmetic composition comprising:
(i) a cosmetic active material
(ii) a continuous phase which comprises at least one water-immiscible liquid carrier oil
(ii) a structurant for the continuous phase
characterised in that the structurant comprises a cyclodipeptide having the general formula 1 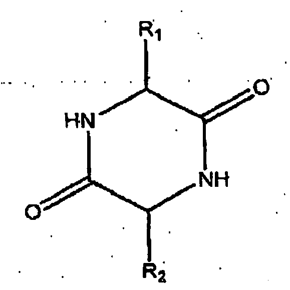 in which at least one of R₁ and R₂ which may be the same or different represents an aliphatic group that is optionally substituted by an aromatic or cycloaliphatic group and the other may alternatively represent hydrogen, the composition contains a water-immiscible aliphatic alcohol which is liquid at 20°C and boils at above 100°C and the continuous phase comprises at least 5% by weight thereof of a monohydric alcohol having at melting point of below 30°C and a boiling point of grater than 100°C, and a water-immiscible carrier cosmetic oil comprising a Silicone oil, and/or non-silicone hydrophobic organic liquid selected from hydrocarbons, hydrophobic aliphatic esters, aromatic esters and hydrophobic ethers.

Such an monohydric alcohol is miscible with commonly employed or contemplated water-immiscible cosmetic oils and therefore be employed to ameliorate the problems identified above, whilst still retaining the benefits from employing such oils.

By employing a monohydric alcohol having the physical attributes identified above as an essential component of the continuous carrier for the cosmetic active, it is possible to render it easier to obtain structured compositions using the selected structural, and can also or alternatively enable compositions, to be obtained in which the problem of undesirable discoloration can be reduced or eliminated. The incorporation of the selected alcohol can lower the temperature at which the desired concentration of structurant dissolves when the carrier also comprises a water-immiscible oil and can also increase the concentration of the cyclic dipeptide which can dissolve in the carrier phase, and furthermore can ameliorate or avoid the problem of the mixture becoming immobile at an excessively high temperatures.

A solution of the structurant in the cyclo dipeptide compound in the monohydric alcohol or its optional mixture with a water-immiscible oil indicates herein that a separate distinct structurant phase is no longer discernible to the human eye.

A composition of this invention will generally be marketed in a container by means of which it can be applied at time of use. This container may be of conventional type.

A second aspect of the invention therefore, provides a cosmetic product comprising a dispensing container having an aperture for delivery of the contents of the container, means for urging the contents of the thorough the said aperture, and a composition of the first aspect of the invention in the container.

Means for urging the contents of the container to the said aperture or apertures, for flow through.them, may be moving parts operable by the user or an orifice in the container opposite the aperture providing digital access.

According to a third aspect of the present invention there is provided a process for the production of a cosmetic composition comprising the steps of:
a) forming a mixture containing a liquid carrier, a structurant dissolved therein, and a solid or a disperse liquid phase comprising cosmetic active in particulate or dissolved form at a temperature of at least 40°C and is above the setting temperature of the mixture;
b) introducing the mixture into a mould which preferably is a dispensing container, and
c) cooling or permitting the mixture to cool to ambient temperature,
characterised in that
the structurant is a cyclo dipeptide that satisfies the general formula 1:- 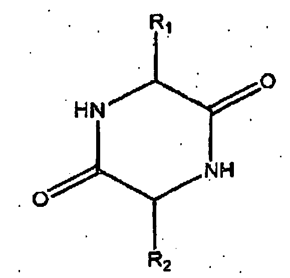 in which at least one of R₁ and R₂ which may be the same or different represents an aliphatic group that is optionally substituted by an aromatic or cycloaliphatic group and the other may alternatively represent hydrogen,
and the carrier comprises at least 5% by weight of a monohydric alcohol having a melting point of below 30°C and a boiling point of greater than 100°C, a cosmetic active material and at least one water-immiscible liquid carrier oil comprising a silicone oil, and/or non-silicone hydrophobic organic liquid selected from hydrocarbons, hydrophobic aliphatic esters, aromatic esters and hydrophobic ethers and the composition contains a water-immiscible aliphatic alcohol which is liquid at 20°C and boils at above 100°C.

A suspended solid may be any cosmetic active that is at least partly insoluble in a lypophilic water-immiscible liquid carrier in the amount incorporated therein and a disperse liquid phase may be a solution of such an active in a hydrophilic or polar solvent.

The cyclo dipeptide may conveniently be dissolved in the monohydric alcohol alone or in the presence of only a fraction of any water-immiscible cosmetic oil. Alternatively, all the cosmetic oil can be present at the dissolution stage. The former process variant is particularly desired.

In a fourth aspect of the present invention, the cosmetic active comprises an antiperspirant or deodorant active. Thus, according to the fourth aspect, there is provided a cosmetic method for preventing or reducing perspiration or odour formation on human skin comprising topically applying to the skin a composition comprising a cosmetic active, a water-immiscible liquid carrier and a structurant compound as defined above in the first aspect in which the cosmetic active is an antiperspirant or deodorant active.

### DETAILED DESCRIPTION AND EMBODIMENTS

The present invention relates to compositions containing a cosmetic active and a water-immiscible phase that is structured with a cyclodipeptide, to a process for their preparation, to their use and to products containing them. Such compositions and the dispensing package will be described in greater detail, including preferences for individual constituents and combinations thereof and preferred process operations.

### Structurant - Cyclo dipeptides

The cyclo dipeptides, sometimes referred to subsequently herein as CDPs, that can be employed in the instant invention can comprise any cycle dipeptide that satisfies general formula 1 above.

It will recognised that the extent to which a CDP is able to structure a water-immiscible carrier liquid or mixture containing it and the properties of the resultant structured material depend upon many factors, including the CDP itself, the chemical nature of the water-immiscible oil or mixture containing it, and the weight ratio of DPD to the oils. For example, different CDPs have different inherent capabilities to structure oils, often manifesting itself in the range of oils which they can structure and/or the long term physical stability of the resultant structured oil and different oils have different inherent propensity to be structured, often manifesting itself in the range of CDPs that can structure them. An increasing ratio of CDP to carrier oil assists in structuring the oil. Structuring herein indicates the formation of a composition having an increased viscosity compared with a corresponding composition free from CDP, but more desirably the CDP/oils and proportions are chosen together such that the composition is gelled at ambient temperature. A gelled composition does not flow within 24 hours out of a filled container of 2 cm diameter that is lain horizontally at 20°C. It can be assessed more quickly as having gelled by employing the test iii) described hereinafter.

In the CDPs herein, R₁ and/or R₂ are desirably linked to the cyclodipeptide nucleus through a methylene group -CH₂-Commonly, R₁ is different from R₂- In many suitable embodiments, one of R₁ and R₂ (the other being H) or more preferably both R₁ and R₂ are selected from aliphatic hydrocarbon groups, preferably saturated, which may be linear or branched, optionally terminating in or substituted by an aryl or cycloaliphatic group, and from aliphatic esters of formula -(CH₂)ₙ-CO₂-R₃ in which in which n is 0 or preferably an integer of at least 1 and R₃ represents an alkyl, cycloalkyl or aryl group. The number of carbons in each of R₁ and R₂ is often selected in the range of from 1 to 35 and in many instances from 1 to 20.

Examples of suitable alkyl groups for R₁ and/or R₂ include ethyl, isopropyl, and isobutyl groups. Others which may be contemplated include 2-ethylbutyl, hexyl, 3-methyl-isononyl, and dodecanyl. Examples of suitable aliphatic ester groups for R₁ and/or R₂ include esters in which n = 0 or 1 or 2, and particularly where n=1. In such or other ester groups, R₃ can represents an alkyl group containing at least 2 carbons, particularly up to 20 carbons, which may be linear or branched, such as an ethyl, isopropyl, isobutyl, 2-ethylbutyl, hexyl, 3-methyl-isononyl, dodecanyl, hexadecanyl or octadecanyl group.

In a number of preferred embodiments, R₃ represents a carbocyclic or heterocyclic group.

In such embodiments, R₃ can comprise two fused rings, but preferably comprises a single six membered ring, either carbocyclic or heterocyclic, or a bridged ring. When R₃ is carbocylic, it can be either saturated or unsaturated, preferably mono- or di-unsaturated or aromatic. When R₃ is heterocyclic, it is preferably saturated.

R₃ is preferably substituted by at least one alkyl substituent, R₄, either directly onto the ring or optionally indirectly via an interposed ether or ester linkage. R₄ preferably contains no more that 19 carbon atoms, such as one having a longest chain length of up to 4 carbon atoms, and/or a total carbon content of up to 5 carbon atoms. R₄ may be linear or branched. Preferred examples include methyl, ethyl, propyl, isopropyl, butyl isobutyl or t-butyl or isopentyl. In a number of very suitable cyclo dipeptides, at least two or more R₄ substituents are present, both or all especially desirably being selected from the above list of preferred examples. The R₄ substituents may be the same, such as two or more methyl substituents, or may be a combination of different substituents such as a methyl and isopropyl substituents. When R₃ is saturated, the R₄ substituents may depend from the same carbon atom in the ring, such as two methyl groups, or from different carbon atoms. In several highly desirable cyclic dipeptides, two alkyl R₄ substituents are meta or para to each other, for example two methyl groups that are meta to each other or a methyl group and an isopropyl group that are para to each other. In yet other cyclo dipeptides, the ring may include a methylene bridge, which preferably likewise completes a six membered ring.

When R₄ is linked to the ring via an ester linkage, the carbonyl carbon in the ester linkage is preferably directly bonded to the ring. In various desirable cyclic dipeptides, R₃ satisfies the formula -Ph-dO-O-R₄ in which R₄ is as described above and particular where R₄ comprises 3 to 6 carbons, such as n-butyl.

When R_{A} is heterocyclic, the heterocyclic atom is suitably nitrogen. Conveniently, the heterocyclic atom can be para to the bond with the cyclo dipeptide residue. Moreover, in a number of desirable cyclo dipeptides, the heteroatom is ortho to at least one alkyl group R₄, better in a saturated ring and especially to up to 4 ortho R₄ groups, that especially are methyl groups,

Examples of such especially preferred R₃ group include thymol, isopinocamphenol and 3,5-diallyl cyclohexanol such as 3,5-dimethyl cyclohexanol.

In several highly desirable embodiments, R₁ represents a benzyl group and R₂ is an ester of formula (CH₂)ₙ-CO₂-R₃ especially those in which n = 1, and R₃ represent a carbocylic or heterocyclic group as described above.

### Continuous Phase - Carrier oils

Herein, the carrier oils include a monohydric alkanol oil together with at least one water-immiscible carrier Cosmetic oil comprising a silicone oil, and/or non-silicone hydrophobic organic liquid selected from hydrocarbons, hydrophobic aliphatic esters, aromatic esters and hydrophobic ethers.

The monohydric alkanol for employment in herein can comprise any alkanol which has a melting point that is no higher than 30°C and a boiling point that is greater than 100°C. Preferred alkanols have a melting point that is below 25°C and especially below 20°C. Preferably, the alkanols have a boiling point that is greater than 120°C and particularly one that is greater than 150°C. Boiling point and melting point data for alkanols is commonly available, or can be readily determined using standard apparatus. Without being prescriptive, alkanols having a suitable melting point and boiling point can be selected from intermediate chain length linear alkanols, such as butanol through to decanol, eg octanol or decanol; or short cycloalkanols such as cyclopentanol through to cycloheptanol, optionally methyl substituted; intermediate or longer chain length branched alkanols, containing for example from 5 to 24 carbons and especially at least 10 carbons, such as secondary aliphatic alcohols, eg isolauryl alcohol isocetyl alcohol isopalmityl alcohol and isostearyl alcohol or secondary alcohols in which the branch contains from 2 to 10 carbons, such as octyl dodecanol. Still other suitable alcohols can be selected from phenyl-terminated short chain aliphatic alcohols, such a benzyl alcohol and phenylethyl alcohol. Mixtures of such alcohols can be employed, both within the sub-classes of alcohols and between the sub-classes.

It is beneficial to choose monohydric alcohols that themselves are comparatively water-immiscible or at best poorly miscible. In practice, the monohydric alcohols above-identified by name normally satisfy such a preference and such a property is ascertainable from standard reference works. Any doubt can be resolved by conducting a simple test. In such a test, preferred alcohols are those which are incapable of forming a stable, single phase when mixed gently with de-ionised water (ie in the absence of any solubilising agent or hydrotrope) at 25°C in a weight ratio of 20 parts alcohol to 80 parts water.

The continuous phase carrier liquid system comprises one or a mixture of materials which are relatively hydrophobic so as to be immiscible in water, in addition to the monohydric alcohol.

The weight proportion of the monohydric alcohol in the carrier liquid is at the discretion of the user. Naturally, it will be understood that the beneficial lowering of the gelation temperature and/or the increase in concentration of structurant that can be incorporated increases non-linearly with the proportion of the monohydric alcohol in the carrier. In practice, the choice of weight proportions takes into account many factors, such as the extent to which a particular CDP suffers from the problems in water-immiscible oils described in the introductory section of this text, and/or which sensory or physical properties are more preferred in the eventual product. The proportion of the monohydric alcohol is normally selected in the range of from 5 to 100% of the weight of the carrier oils. In many embodiments, its weight proportion is at least 20%. To permit significant variation in the sensory properties of the eventual composition, the weight proportion of the monohydric alcohol conveniently is not more than 70% or 80% of the carrier oils.

The more the monohydric alcohol that is present, the greater the extent to which it can enhance the solubility of the CDP and lower the gelling temperature of the liquid carrier. In many compositions according to the present invention, the selected monohydric alcohol is present in a weight ratio to the CDP of at least 1:1, particularly at least 2:1 and in many practical embodiments is at least 4:1. Although a weight ratio to the CDP of greater than 100:1 can be contemplated, the weight ratio is normally up to 100:1, and in many instances is up to 70:1. In various practical embodiments, the weight ratio to CDP is up to 20:1.

Some hydrophilic liquid may be included in the carrier, provided the overall carrier liquid mixture is immiscible with water. It will generally be desired that this carrier is liquid (in the absence of structurant) at temperatures of 15°C and above. It may have some volatility but its vapour pressure will generally be less than 4kPa (30 mmHg) at 25°C so that the material can be referred to as an oil or mixture of oils. More specifically, it is desirable that at least 80% by weight of the hydrophobic carrier liquid should consist of materials with a vapour pressure not over this value of 4kPa at 25°C.

It is preferred that the hydrophobic carrier material includes a volatile liquid silicone, i.e. liquid polyorganosiloxane. To class as "volatile" such material should have a measurable vapour pressure at 20 or 25°C.. Typically the vapour pressure of a volatile silicone lies in a range from 1 or 10 Pa to 2 kPa at 25°C.

It is desirable to include volatile silicone because it gives a "drier" feel to the applied film after the composition is applied to skin.

Volatile polyorganosiloxanes can be linear or cyclic or mixtures thereof. Preferred cyclic siloxanes include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below 10⁻⁵ m²/sec (10 centistokes), and particularly above 10⁻⁷ m²/sec (0.1 centistokes), the linear siloxanes normally exhibiting a viscosity of below 5 x 10⁻⁶ m²/sec (5 centistokes). The volatile silicones can also comprise branched linear or cyclic siloxanes such as the aforementioned linear or cyclic siloxanes substituted by one or more pendant -0-Si(CH₃)₃ groups. Examples of commercially available silicone oils include oils having grade designations 344, 345, 244, 245 and 246 from Dow Corning Corporation; Silicone 7207 and Silicone 7158 from Union Carbide Corporation; and SF1202 from General Electric.

The hydrophobic carrier employed in compositions herein can alternatively or additionally comprise non-volatile silicone oils, which include polyalkyl siloxanes, polyalkylaryl siloxanes and polyethersiloxane copolymers. These can suitably be selected from dimethicone and dimethicone copolyols. Commercially available non-volatile silicone oils include products available under the trademarks Dow Corning 556 and Dow Corning 200 series. Other non volatile silicone oils include that bearing the trademark DC704. Incorporation of at least some non-volatile silicone oil having a high refractive index such as of above 1.5, eg at least 10% by weight (preferably at least 25% to 100% and particularly from 40 to 80%) of the silicone oils is often beneficial in some compositions, because this renders it easier to match the refractive index of the constituents of the composition and thereby easier to produce transparent or translucent formulations.

The water-immiscible oil employed in the carrier in addition to the monohydric alcohol may comprise from 0% to 100% by weight of one or more liquid silicones. In some embodiments, there is sufficient liquid silicone to provide at least 10%, better at least 15%, by weight of the whole composition. When silicone oil is used in various embodiments, for example in emulsions, volatile silicone preferably constitutes from 20 to 100% of the weight of the carrier liquid. In a number of embodiments, when a non-volatile silicone oil is present, its weight ratio to volatile silicone oil is chosen in the range of from 5:1 to 1:50.

Silicon-free hydrophobic oils can be used instead of, or more preferably in addition to liquid silicones. Silicon-free hydrophobic organic oils that can be incorporated include liquid aliphatic hydrocarbons such as mineral oils or hydrogenated polyisobutene, often selected to exhibit a low viscosity. Further examples of liquid hydrocarbons are polydecene and paraffins and isoparaffins of at least 10 carbon atoms.

Other suitable hydrophobic carriers comprise liquid aliphatic or aromatic esters. Suitable aliphatic esters contain at least one long chain alkyl group, such as esters derived from C₁ to C₂₀ alkanols esterified with a C₈ to C₂₂ alkanoic acid or C₆ to C₁₀ alkanedioic acid. The alkanol and acid moieties or mixtures thereof are preferably selected such that they each have a melting point of below 20°C. These esters include isopropyl myristate, lauryl myristate, isopropyl palmitate, di-isopropyl sebacate and di-isopropyl adipate.

Suitable liquid aromatic esters, preferably having a melting point of below 20°C, include fatty alkyl benzoates. Examples of such esters include suitable C₈ to C₁₈ alkyl benzoates or mixtures thereof, including in particular C₁₂ to C₁₅ alkyl benzoates eg those available under the trademark Finsolv. Other suitable aromatic esters include alkyl naphthalates, alkyl salicylates and aryl benzoates of MP <20°C. Incorporation of such aromatic esters as at least a fraction of the hydrophobic carrier liquid can be advantageous, because they can raise the average refractive index of volatile-silicone-containing carriers, and thereby render it easier to obtain translucent or transparent formulations.

Further instances of suitable hydrophobic carriers comprise liquid aliphatic ethers derived from at least one fatty alcohol, such as myristyl ether derivatives e.g. PPG-3 myristyl ether or lower alkyl ethers of polygylcols such as an ether having named as PPG-14 butyl ether by the CTFA.

Aliphatic alcohols which are liquid at 20°C and boil at above 100°C provide an essential constituent of the instant invention formulations, and especially desirably those which are clearly water-immiscible. Such alcohols can often constitute from 10% or 15% to 30% or 55% by weight of the cosmetic composition.

Silicon-free liquids can constitute from 0-100% of the residue of the water-immiscible liquid carrier, i.e. other than said above-mentioned essential aliphatic alcohols, but it is preferred that-silicone oil is present and that the amount of silicon-free constituents preferably constitutes up to 50 or 60% and in many instances from 10 to 60% by weight, eg 15 to 30% or 30 to 50% by weighty of the water-immiscible carrier liquid.

As will be explained in more detail below, in cosmetic compositions herein, the structured water-immiscible carrier liquid may be the continuous phase in the presence of a dispersed second phase, which may comprise a suspension of particulate solid forming a suspension stick or a dispersion of droplets of a lypophobic liquid. Such a solid may be a particulate antiperspirant or deodorant active or pigment. Such a disperse liquid phase may comprise a solution of the aforementioned active or actives in water or other hydrophilic ie lypophobic solvent.

As mentioned hereinabove, in accordance with the first aspect, the invention requires a CDP structurant to structure a carrier oil that comprises a monohydric alcohol. A cosmetic active is present in cosmetic compositions and other materials may also be present depending on the nature of the composition. The various materials will now be discussed by turn and some preferred features and possibilities will be indicated.

The proportion of the CDP structurant in a composition of this invention is likely to be from 0.1 to 15% by weight of the whole composition and preferably from 0.1 up to 10%. Its weight proportion more commonly is at least 0.3% and in many instances not more than 5%. In some especially desirable embodiments, the amount of CDP structurant is from 0.5% to 3.5% or 5%. It will be recognised that for any particular CDP, its maximum proportion in the composition will vary in accordance with its solubility in the selected monohydric alcohol and the conditions prevailing during the dissolution process, such as temperature. Herein, unless other wise stated, a % for the CDP is by weight based on the entire composition.

If the composition is an emulsion with a separate disperse phase, the amount of structurant compound(s) is likely to be from 0.3 to 20% by weight of the continuous phase, more likely from 0.6% to 8% of this phase. In some highly desirable embodiments the hydrophobic carrier continuous phase contains from 2 to 5% by weight of the CDP.

### Liquid Disperse Phase in emulsions

If the composition is an emulsion in which the cyclo dipeptide acts as a structurant in the hydrophobic continuous phase, the emulsion will contain a more polar or lypophobic disperse phase. The disperse phase may be a solution of an active ingredient.

The hydrophilic disperse phase in an emulsion commonly comprises water as solvent and can comprise one or more water soluble or water miscible liquids in addition to or in replacement of water. The proportion of water in an emulsion according to the present invention is often selected in the range of up to 60%, and particularly from 10% up to 40% or 50% of the whole formulation.

One class of water soluble or water-miscible liquids comprises short chain monohydric alcohols, e.g. C₁ to C₄ and especially ethanol or isopropanol, which can impart a deodorising capability to the formulation. Ethanol gives a cooling effect on application to skin, because it is very volatile. It is preferred that the content of ethanol or any other monohydric alcohol with a vapour pressure above 1.3kPa (10 mmHg) is not over 15% better not over 8% by weight of the composition.

A further class of hydrophilic liquids comprises diols or polyols preferably having a melting point of below 40°C, or which are water miscible. Examples of water-soluble or water-miscible liquids with at least one free hydroxy group include ethylene glycol, 1,2-propylene glycol, 1,3-butylene glycol, hexylene glycol, diethylene glycol, dipropylene glycol, 2-ethoxyethanol, diethylene glycol monomethylether, triethyleneglycol monomethylether and sorbitol. Especially preferred are propylene glycol and glycerol.

In an emulsion the disperse phase is likely to constitute from 5 to 80 or 85% of the weight of the composition preferably from 5 to 50 or 65% more preferably from 25 or 35% up to 50 or 65%, while the continuous phase with the structurant therein provides the balance from 15 or 35% up to 95% of the weight of the composition. Compositions with high proportion of disperse phase, i.e. from 65 to 85% disperse phase, may be advantageous because they can give good hardness even though the concentration of structurant may be only a small percentage of the total composition. However, compositions with a lower proportion of disperse phase can also be advantageous because they tend to offer a drier and warmer feel.

An emulsion composition will generally include one or more emulsifying surfactants which may be anionic, cationic, zwitterionic and/or nonionic surfactants. The proportion of emulsifier in the composition is often selected in the range up to 10% by weight and in many instances from 0.1 or 0.25 up to 5% by weight of the composition. Most preferred is an amount from 0.1 or 0.25 up to 3% by weight. Nonionic emulsifiers are frequently classified by HLB value. It is desirable to use an emulsifier or a mixture of emulsifiers with an overall HLB value in a range from 2 to 10 preferably from 3 to 8.

It may be convenient to use a combination of two or more emulsifiers which have different HLB values above and below the desired value. By employing the two emulsifiers together in appropriate ratio, it is readily feasible to attain a weighted average HLB value that promotes the formation of an emulsion.

Many suitable emulsifiers of high HLB are nonionic ester or ether emulsifiers comprising a polyoxyalkylene moiety, especially a polyoxyethylene moiety, often containing from about 2 to 80, and especially 5 to 60 oxyethylene units, and/or contain a polyhydroxy compound such as glycerol or sorbitol or other alditol as hydrophilic moiety. The hydrophilic moiety can contain polyoxypropylene. The emulsifiers additionally contain a hydrophobic alkyl, alkenyl or aralkyl moiety, normally containing from about 8 to 50 carbons and particularly from 10 to 30 carbons. The hydrophobic moiety can be either linear or branched and is often saturated, though it can be unsaturated, and is optionally fluorinated. The hydrophobic moiety can comprise a mixture of chain lengths, for example those deriving from tallow, lard, palm oil, sunflower seed oil or soya bean oil. Such nonionic surfactants can also be derived from a polyhydroxy compound such as glycerol or sorbitol or other alditols. Examples of emulsifiers include ceteareth-10 to - 25, ceteth-10-25, steareth-10-25 (i.e. C₁₆ to C₁₈ alcohols ethoxylated with 10 to 25 ethylene oxide residues) and PEG-15-25 stearate or distearate. Other suitable examples include C₁₀-C₂₀ fatty acid mono, di or tri-glycerides. Further examples include C₁₈-C₂₂ fatty alcohol ethers of polyethylene oxides (8 to 12 EO).

Examples of emulsifiers, which typically have a low HLB value, often a value from 2 to 6 are fatty acid mono or possibly diesters of polyhydric alcohols such as glycerol, sorbitol, erythritol or trimethylolpropane. The fatty acyl moiety is often from C₁₄ to C₂₂ and is saturated in many instances, including cetyl, stearyl, arachidyl and behenyl. Examples include monoglycerides of palmitic or stearic acid, sorbitol mono or diesters of myristic, palmitic or stearic acid, and trimethylolpropane monoesters of stearic acid.

A particularly desirable class of emulsifiers comprises dimethicone copolymers, namely polyoxyalkylene modified dimethylpolysiloxanes. The polyoxyalkylene group is often a polyoxyethylene (POE) or polyoxypropylene (POP) or a copolymer of POE and POP. The copolymers often terminate in C₁ to C₁₂ alkyl groups.

Suitable emulsifiers and co-emulsifiers are widely available under many trade names and designations including Abil^{™}, Arlacel^{™}, Brimj^{™}, Cremophor^{™}, Dehydrol^{™}, Dehymuls^{™}, Everest^{™}, Lameform^{™}, Pluronic^{™}, Prisorine^{™}, Quest PGPH^{™}, Span^{™}, Tween^{™}, SF1228, DC3225C and Q2-5200.

### Cosmetic Actives

The cosmetic actives employable herein can comprise antiperspirant or deodorant actives or pigments.

### Antiperspirant Actives

The composition preferably contains an antiperspirant active. Antiperspirant actives, are preferably incorporated in an amount of from 0.5-60%, particularly from 5 to 30% or 40% and especially from 5 or 10% to 30 or 35% of the weight of the composition.

Antiperspirant actives for use herein are often selected from astringent active salts, including in particular aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates and activated aluminium chlorohydrates.

Aluminium halohydrates are usually defined by the general formula Al₂(OH)ₓQ_{y}.wH₂0 in which Q represents chlorine, bromine or iodine, x is variable from 2 to 5 and x + y = 6 while wH₂O represents a variable amount of hydration. Especially effective aluminium halohydrate salts, known as activated aluminium chlorohydrates, are described in EP-A-6739 (Unilever NV et al). Some activated salts do not retain their enhanced activity in the presence of water but are useful in substantially anhydrous formulations, i.e. formulations which do not contain a distinct aqueous phase.

Zirconium actives can usually be represented by the empirical general formula: ZrO(OH)_{2n-nz}Bz.wH₂0 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂0. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂ (NH₂) COOH.

It is highly desirable to employ complexes of a combination of aluminium halohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

Other actives which may be utilised include astringent titanium salts, for example those described in GB 2299506A.

The proportion of solid antiperspirant salt in a suspension composition normally includes the weight of any water of hydration and any complexing agent that may also be present in the solid active. However, when the active salt is incorporated in solution in a hydrophilic solvent such as a glycol, its weight commonly excludes any water present.

If the composition is in the form of an emulsion the antiperspirant active will be dissolved in the disperse phase. In this case, the antiperspirant active will often provide from 3 to 60% by weight of the disperse phase, particularly from 10% or 20% up to 55% or 60% of that phase. Alternatively, the composition may take the form of a suspension in which antiperspirant active in particulate form is suspended in the water-immiscible liquid carrier. Such a composition will probably not have any separate aqueous phase present and may conveniently be referred to as "substantially anhydrous" although it should be understood that some water may be present bound to the antiperspirant active or as a small amount of solute within the water-immiscible liquid phase. In such compositions, the particle size of the antiperspirant salts often falls within the range of 0.1 to 200 µm with a mean particle size often from 3 to 20µm. Both larger and smaller mean particle sizes can also be contemplated such as from 20 to 50µm or 0.1 to 3µm.

### Deodorant Actives

Suitable deodorant actives can comprise deodorant effective concentrations of antiperspirant metal salts, deoperfumes, and/or microbicides, including particularly bactericides, such as chlorinated aromatics, including biguanide derivatives, of which materials known as Igasan DP300^{™} (triclosan), Tricloban^{™}, and Chlorhexidine warrant specific mention. A yet another class comprises biguanide salts such as are available under the trade mark Cosmosil^{™}. Deodorant actives are commonly employed at a concentration of from 0.1 to 25% by weight.

### Optional ingredients

Other optional ingredients include wash-off agents, often present in an amount of up to 10% w/w to assist in the removal of the formulation from skin or clothing. Such wash-off agents are typically nonionic surfactants such as esters or ethers containing a C₈ to C₂₂ alkyl moiety and a hydrophilic moiety which can comprise a polyoxyalkylene group (POE or POP) and/or a polyol. A further optional constituent of the formulation comprises one or more further structurants which can be employed in addition to the cyclo dipeptide. Herein, the CDP may be the primary structurant, by which is meant that is employed at a concentration that is higher than that of the further structurant. However, in some advantageous embodiments, the further structurant may be present in an amount that is at least that of the CDP. In such advantageous embodiments, the CDP is acting to moderate the properties of the further structurant such that the properties using the combined structurant system are superior in at least one desirable respect to using the further structurant alone. The amount of such further structurants in the formulation is often from zero to not more than 15% of the formulation. In some instances, the further structurant is present in a weight ratio to the CDP of from 10:1 to 1:10.

The further structurants employable herein can be non-polymeric or polymeric. Solid linear fatty alcohol and/or a wax may be included but are not preferred. In anhydrous compositions notably antiperspirants which are suspension sticks, non-polymeric further structurants, sometimes referred to as gellants, can be selected from fatty acids or salts thereof, such as stearic acid or sodium stearate or 12-hydroxy stearic acid. Linear fatty acids are preferably not used in aqueous sticks, e.g. aqueous emulsion sticks because they can form insoluble precipitates with aluminium ions. Other suitable gellants can comprise dibenzylidene alditols, e.g. dibenzylidene sorbitol. Further suitable gellants can comprise selected N-acyl amino acid derivatives, including ester and amide derivatives, such as N-lauroyl glutamic acid dibutylamide, which gellants can be contemplated in conjunction with 12-hydroxy stearic acid or an ester or amide derivative thereof. Still further gellants include amide derivatives of di or tribasic carboxylic acids, such as alkyl N,N' dialkylsuccinamides, e.g. dodecyl N,N'-dibutylsuccinamide. When employing further structurants comprising N-acyl amino acid derivatives, in some highly desirably formulations their weight ratio to CDP is selected in the range of 1:1 to 6:1.

Polymeric structurants which can be employed as further structurants can comprise organo polysiloxane elastomers such as reaction products of a vinyl terminated polysiloxane and a cross linking agent or alkyl or alkyl polyoxyalkylene-terminated poly (methyl substituted) or poly (phenyl substituted) siloxanes. A number of polyamides have also been disclosed as structurants for hydrophobic liquids. Polymers containing both siloxane and hydrogen bonding groups, which might be used as secondary structurants, have been disclosed in WO 97/36572 and WO 99/06473. If an aqueous disperse phase is present, polyacrylamides, polyacrylates or polyalkylene oxides may be used to structure or thicken this aqueous phase.

It is highly desirable that any further structurant employed herein is itself fibre-forming, that is to say forms a fibrous structure within the hydrophobic phase. Most preferably the fibre-forming structurant is one in which the fibrous structure is not visible to the human eye.

Fatty alcohols which are solid at room temperature of 20°C, such as linear monohydric alkanols containing at least 12 carbons e.g. stearyl alcohol or behenyl alcohol, lead to deposits with an opaque white appearance and are preferably substantially absent, by which we mean present in an amount of no more than 3% by weight of the composition, more preferably less than 1% and most preferably are not incorporated specifically, ie 0%. As already mentioned, fatty alcohols are often regarded as coming within the general category of waxy materials. More generally the term "wax" is conventionally applied to a variety of materials and mixtures (including some fatty alcohols) which have some diversity in chemical structure but similarity in physical properties. The term generally denotes materials which are solid at 30°C, often also solid up to 40°C, having a waxy appearance or feel, but which gradually soften and eventually melt to a mobile liquid at a temperature below 95°C usually below 90°C.

Possibly the composition does not include more than 3% of any material which is a wax, ie a solid at 30°C but softens at an elevated temperature and at 95°C is molten and soluble in the water-immiscible liquid, yet which is unable to form a network of fibres therein on cooling to 20°C.

The compositions herein can incorporate one or more cosmetic adjuncts in amounts conventionally contemplatable for cosmetic solids or soft solids. Such cosmetic adjuncts can include skin feel improvers, such as small particle inorganic mineral substances like talc, finely divided silica and/or bentonite or similar clays, or finely divided polyethylene, for example in an amount of up to about 10%; skin benefit agents such as allantoin or lipids, for example in an amount of up to 5%; colours; skin cooling agents other than the already mentioned alcohols, such a menthol and menthol derivatives, often in an amount of up to 2%, all of these percentages being by weight of the composition. A commonly employed adjunct is a perfume, which is normally present at a concentration of from 0 to 4% and in many formulations from 0.25 to 2% by weight of the composition.

### Product Form

The sticks produced employing the CDP structurants can be either opaque or translucent or even transparent, depending at least partly on the extent to which the refractive indices (RI) of the appropriate ingredients are matched. Translucent or transparent formulations are possible in respect of the invention formulations because the CDP structurant forms a fibrous structure within the liquid hydrophobic carrier that is not seen by the human eye. By matched herein is meant that the difference between the refractive indices is less than 0.005 and preferably less than 0.002. In suspension sticks, to achieve at least translucency without using exclusively sub-micron sized particles, it is necessary to match the RI of the suspended cosmetic active, eg the particulate antiperspirant salt, with the RI of the suspending carrier oil mixture. This can be assisted by a suitable choice of oils, and in particular mixtures containing those having an RI of above 1.46, such as from 1.46 to 1.56. In regard to the suspended particulates, RI matching can be assisted by controlling the particle size distribution, and particularly by not permitting an excess proportion of 1 to 10 micron particles and advantageously by avoiding the manufacture of hollow sphere antiperspirant actives or subsequently removing the hollows. Matching can be further assisted by modifying the RI of the suspended cosmetic active, such as an aluminium-containing antiperspirant active by post treating it with water (re-hydration) or by retaining a comparatively high water content during the manufacture process. In emulsion formulations, the relevant ingredients to RI match comprise the disperse and continuous liquid phases.

It is highly desirable to employ RI matching as indicated above in conjunction with the exclusion, to the extent necessary, of additional suspended materials having a different refractive index from the suspending medium, such as for example a suspended filler or additional cosmetic active, to enable the resultant composition to transmit at least 1% light (in the test described hereinafter).

### Mechanical Properties and Product Packages

The compositions of this invention are structured liquids and are firm in appearance. A composition of this invention will usually be marketed as a product comprising a container with a quantity of the composition therein, where the container has an aperture for the delivery of composition, and means for urging the composition in the container towards the delivery aperture. Conventional containers take the form of a barrel of oval cross section with the delivery aperture at one end of the barrel.

A composition of this invention may be sufficiently rigid that it is not apparently deformable by hand pressure and is suitable for use as a stick product in which a quantity of the composition in the form of a stick is accommodated within a container barrel having an open end at which an end portion of the stick of composition is exposed for use. The opposite end of the barrel is often closed.

Generally the container will include a cap for its open end and a component part which is sometimes referred to as an elevator or piston fitting within the barrel and capable of relative axial movement along it. The stick of composition is accommodated in the barrel between the piston and the open end of the barrel. The piston is used to urge the stick of composition along the barrel. The piston and stick of composition may be moved axially along the barrel by manual pressure on the underside of the piston using a finger or rod inserted within the barrel. Another possibility is that a rod attached to the piston projects through a slot or slots in the barrel and is used to move the piston and stick. Preferably the container also includes a transport mechanism for moving the piston comprising a threaded rod which extends axially into the stick through a correspondingly threaded aperture in the piston, and means mounted on the barrel for rotating the rod. Conveniently the rod is rotated by means of a handwheel mounted on the barrel at its closed end, i.e. the opposite end to the delivery opening.

The component parts of such containers are often made from thermoplastic materials, for example polypropylene or polyethylene. Descriptions of suitable containers, some of which include further features, are found in US patents 4865231, 5000356 and 5573341.

### Composition Preparation

Compositions of this invention can be produced by process similar to conventional processes for making cosmetic solids. Such processes involve forming a heated mixture of the structurant in the carrier oil, in this case the monohydric alcohol and optionally together with a fraction or even all of any other oil, at a temperature which is sufficiently elevated that all the structurant dissolves, pouring that mixture into a mould, which may take the form of a dispensing container, and then cooling the mixture whereupon the structurant solidifies into a network of fibres extending through the water-immiscible liquid phase. The employment of the monohydric alcohol in the continuous carrier enables the benefits of higher CDP concentration and reduced gelling temperature to be attained

A convenient process sequence for a composition which is a suspension comprises first forming a solution of the structurant in the monohydric alcohol and optionally a fraction of or even all the water-immiscible liquids. This is normally carried out by agitating the mixture at a temperature sufficiently high that all the structurant dissolves (the dissolution temperature) such as a temperature in a range from 50 to 140°C. Thereafter, the particulate constituent, for example particulate antiperspirant active, is blended with the hot mixture. This may be done slowly, and/or the particulate solid preheated, in order to avoid premature gelation. The resulting blend is then introduced into a dispensing container such as a stick barrel. This is usually carried out at a temperature 5 to 30°C above the setting (gelling) temperature of the composition. The container and contents are then cooled to ambient temperature. Cooling may be brought about by nothing more than allowing the container and contents to cool. Cooling may be assisted by blowing ambient or even refrigerated air over the containers and their contents.

In a suitable procedure for making emulsion formulations, a solution of the structurant in the continuous carrier phase is prepared at an elevated temperature just as for suspension sticks. If any emulsifier is being used, this is conveniently mixed into this liquid phase. Separately, an aqueous or hydrophilic disperse phase is prepared by introduction of antiperspirant active into the liquid part of that phase (if this is necessary: antiperspirant actives can sometime be supplied in aqueous solution which can be utilised as is). If possible, this solution of antiperspirant active which will become the disperse phase is preferably heated to a temperature similar to that of the continuous phase with structurant therein, but without exceeding the boiling point of the solution, and then mixed with the continuous phase. Alternatively, the solution is introduced at a rate which maintains the temperature of the mixture. If it is necessary to work at a temperature above the boiling temperature of the disperse phase, or at a temperature where evaporation from this phase is significant, a pressurised apparatus could be used to allow a higher temperature to be reached. After the two phases are mixed, the resulting mixture is filled into dispensing containers, typically at a temperature 5 to 30°C above the setting temperature of the composition, and allowed to cool as described above for suspension sticks.

The cosmetic compositions according to the present invention employ a mixture of at least one hydrophobic cosmetic oil (carrier fluid) with the monohydric alcohol. In some convenient preparative routes, it is desirable to dissolve the CDP structurant in the alcohol, optionally in conjunction with a minor proportion of an alcohol having some water-miscibility.and boiling point above the dissolution temperature of CDP in the alcoholic fluid. This enables the remainder of the carrier fluids to avoid being taken to the temperature at which the CDP dissolves or melts.. The proportion of the carrier fluids for dissolving the CDP is often from 15 to 85% by weight of the carrier fluids, and particularly from 20 to 40% or 70%. In one variation, the CDP structurant is first dissolved by heating to an elevated temperature with stirring in a mixture comprising the monohydric alcohol plus up to a quarter or even up to half of the total of any water-immiscible cosmetic oil employed in the composition, and there after mixing the solution of the CDP with the remainder of the cosmetic oil and the cosmetic active, such a particulate antiperspirant or an aqueous solution of an Antiperspirant. The remainder of the cosmetic oil and of the cosmetic active are taken to a suitable temperature such that the temperature of their mixture with the CDP solution is still above the gelling temperature of the composition, preferably no more than 5 or 10°C above the gelling temperature, which may have been determined in a previous trial.

### Structurant Preparation

CDP structurants can be made by one or more of the general preparative routes published in the above-identified papers by Hanabusa with appropriately chosen reagents to obtain the desired substituent groups R₁ and R₂ of the cyclo dipeptide, and/or by variations described hereinbelow or the general method described herein to make the materials CDP1 to CDP13.

One route of general applicability described by Hanabusa comprises the cyclisation of dipeptide ethyl esters under reflux in 1,3,5-trimethyl benzene, the esters being obtained by catalytic hydrogenation of the corresponding N-benzoylcarbonyl dipeptide ethyl ether with 10% Pd-C. In a variation thereof, ester groups in an existing ester CDP can be replaced in a conventional transesterification process in the corresponding alcohol, eg 3,7-dimethyloctanol.

Various of the CDPs are derivable indirectly from aspartame by esterifying cyclo[(R)-phenylalanyl] that is obtainable by heating aspartame, preferably in the presence of a substantial excess of a low molecular weight aliphatic alcohol, such as isopropanol, under reflux for a long period. Desirably, the alcohol is employed in a weight ratio to aspartame of greater than 50:1 such as up to 100:1, and the reaction is continued for at least 10 hours at reflux temperature, such as from 15 to 24 hours. During the reaction, the aspartame gradually dissolves. On cooling, the resultant solution yields a white powder. Removal of the solvent from the filtrate yields a solid which, after washing with acetone, provides a further amount of the white product, confirmed by a combined yield of the CDP precursor acid of 79 %.

The precursor acid can be reacted with the relevant alcohol of formula R_{A}OH, preferably in a mole ratio to the precursor of at least 1:1 to 10:1, particularly from 1.5:1 to 7:1 and especially at least 2:1 in dimethyl sulphoxide, conveniently in a ratio of at least 4:1 (vol:wt), preferably from 6:1 to 12:1, and preferably in the presence of a promoter, such as a carbonyldiimidazole, in an amount preferably from 0.5 to 2 moles of promoter per mole of precursor acid. The reaction is conveniently carried out at a mildly elevated temperature, such as up to 60°C and particularly from 40 to 60°C for a period of at least 6 hours and preferably from 9 to 24 hours. The resultant solution is quenched in excess ambient or cooler water, desirably after the solution has cooled to ambient, a solid precipitates and is filtered off, water washed until no residual diimidazole remained and then can be purified by washing with diethyl ether or toluene, and dried.

### Examples

### Preparation of CDP Structurants

The cyclo dipeptide structurants employed in the following Examples and Comparisons were made by the following general method employing (2S-cis)-(-)-5-benzyl-3,6-dioxo-2-piperazine acetic acid (DOPAA) which was reacted with the alcohols specified in Table 1 below.

A 250 ml 3 necked round bottomed flask equipped with a stirrer was charged with (2S-cis)-(-)-5-benzyl-3,6-dioxo-2-piperazine acetic acid (DOPAA) (18.4 mmol), and dimethyl sulfoxide (8mls per 1g of DOPAA) was then introduced at laboratory ambient temperature (about 22°C) with stirring. The DOPAA dissolved only partially. 1,1'-carbonyldiimidazole (22 mmol) was then introduced with stirring in the amount specified in the Table. Vigorous effervescence occurred and the react mixture was left stirring at room temperature for 45 minutes after which time the reaction mixture went clear. The specified alcohol (92 mmol) was stirred into the clear reaction mixture and maintained at 50°C overnight (between 16 and 20 hours), whereupon it was allowed to cool to ambient temperature (about 22°C), and poured into water, producing a precipitate which was filtered off and washed with further quantities of water until any residual diimidazole had been removed (as shown by ¹Hnmr). The washed precipitate was then washed with diethyl ether. The washed product was dried in a vacuum oven to constant weight and its melting point determined, the results quoted herein being obtained by DSC with a heating rate of 10°C/min, except for those marked ^{ET}, which were obtained using a an Electrothermal 9109 digital melting point measuring apparatus.

**Table 1**

| CDP | Alcohol | Purity % | Melting Point °C |
|---|---|---|---|
| CDP1 | (1S, 2R, 5S)-(+) Menthol | 98.7 | 238 |
| CDP2 | Thymol | 99.3 | 212 |
| CDP3 | 1R, 2R, 3R, 5S-(-)-iso-pinocamphenol | 68 | >200 |
| CDP4 | 3,5-dimethyl-cyclohexanol | 94 | 212 |
| CDP5 | phenol | 99.7 | 246 |
| CDP6 | butyl-4-hydroxy benzoate | 98.5 | 217 |
| CDP7 | iso-propanol | 98.5 | 215 |
| CDP8 | n-propanol | 98.2 | >200 |
| CDP9 | 4-t-butylphenol | 99.1 | 237 |
| CDP10 | carveol | 65.0 | 215^{ET} |
| CDP11 | carvacrol | 99.1 | 229^{ET} |
| CDP12 | 5,6,7,8-tetrahydronaphth-2-ol | 99.3 | 220^{ET} |
| CDP13 | 2-isopropoxyphenol | 98.8 | 178^{ET} |

### Materials

The materials used in gel studies or the preparation of cosmetic formulations, and their proprietary names, other than the structurants CDP1 to CDP9, are summarised in Table 2:

**Table 2**

| | Abrev | CFTA name | Trade Name/supplier |
|---|---|---|---|
| Monohydric Alcohols | | | |
| 1 | ISA | Isostearyl Alcohol | Pricerine 3515^{™} - Uniqema |
| 2 | ODA | Octyl Dodecanol | Eutanol G^{™} - Cognis |
| 3 | BMA | Benzyl Alcohol | Acros |
| 4 | 8MA | octyl Alcohol | Sigma |
| 5 | 10MA | decyl Alcohol | Sigma |
| 6 | ICA | iso-cetyl Alcohol | Eutanol G16 - Cognis |

| Water-immiscible oil | | | |
|---|---|---|---|
| 7 | TN | C₁₂-₁₅ alkyl benzoate | Finsolv TN ^{™} from Finetex Inc |
| 8 | 245 | Cyclomethicone | DC 245 ^{™} - Dow Corning Inc |
| 9 | 364 | Hydogenated Polydecene | Silkflo 364 NF ^{™}- Albemarle |
| 10 | 704 | 1,1,5,5-tetraphenyl trisiloxane | DC704^{™}: Dow Corning Inc |

| Auxiliary Structurant | | | |
|---|---|---|---|
| 11 | GP1 | N-lauroyl-L-glutamic acid Di-n-butylamide | GP-1^{™}Ajinomoto Co Inc |
| 18 | DBS | dibenzylidene sorbitol | Roquette |
| 19 | HSA | 12- hydroxystearic acid | 12-HSA (CasChem) |
| 20 | 930 | Polyamide | Versamid 930^{™} (Cognis) |

| Emulsifier | | | |
|---|---|---|---|
| 12 | EM90 | Dimethicone Copolyol | Abil EM90^{™} -Th. Goldschmidt AG |
| 21 | P135 | dipolyhydroxy-stearate | Arlacel P135^{™} (Uniquema) |

| Cosmetic Active | | | |
|---|---|---|---|
| 13 | R908 | Al/Zr Tetrachlorohydrex glycine complex | Reach 908^{™} - Reheis Inc |
| 14 | A418 | Milled Macrospherical AACH | A418^{™} - Summit |
| 15 | Z50 | 50% aqueous solution of Al/Zr pentachlorohydrate | Zirconal 50^{™} - BK Giulini |
| 16 | R67* | Water-modified AZAG | Rezal 67^{™} modified in-house |
| 22 | 36GP | solid Al/Zr tetra-chlorhydrex glycine | Rezal 36GP^{™} Reheis Inc |
| 23 | P5G | Al/Zr pentachloro-hydrex glycine complex (RI 1.530) | P5G^{™} (BK Giulini) |

| Water-miscible liquids | | | |
|---|---|---|---|
| 17 | GOH | Glycerin | Glycerol - Aldrich |
| 24 | PG | propane-1,2-diol | Fisher |
| 25 | DPG | di(propane-1,2-diol) | Fisher |
| 26 | TPnB | tri(1,2-propane-Dowanol diol) n-butylether | TPnB^{™} - Dow Corning Inc |

| Other Ingredients | | | |
|---|---|---|---|
| 27 | H30 | silica | H30^{™} - Wacker-Chemie GmbH |
| 28 | H30R X | silica | H30RX^{™} - Wacker-Chemie GmbH |
| 29 | H18 | silica | H18^{™} - Wacker-Chemie GmbH |

| | | | |
|---|---|---|---|
| 16 - R67* was made in house by freeze drying an AZAG solution (Rezal 67^{™}) and sieving to obtain particulate solid free from hollow particles (-37% of particles <10µm) and water treated to RI =1.526. 23 - P5G was free from hollow particles and contained -25% particles of <10µM). | | | |

### Example 1 - Structured Gels

In this Example, gels were made or attempted to be made in a number of representative organic solvents, using the structurants CDP1 to CDP13.

The gels were prepared in 30ml clear glass bottles. The solvent and gelling agent were weighed directly into the bottle to give a total mixture weight of 10g. A small Teflon^{™} stirrer bar was placed in the bottle and the mixture stirred and heated until the cyclo dipeptide had dissolved. The bottle was then removed from the heat and the solution allowed to cool and gel under quiescent conditions.

The ease of gel formation was assessed by determining for each of the cosmetic base formulations the temperature at which the CDP structurant dissolved in the chosen oil(s) and if dissolution was.observed, the temperature at which a gel formed on cooling the formulation. The results are summarised in Table 3.

These Examples and comparisons demonstrate the relative ease or difficulty of forming gelled cosmetic base formulations, depending on which oils are employed during the dissolution of the CDP structurant and the subsequent formation of a gel as the compositions cools. The results are representative of corresponding formulations in which a cosmetic active is also introduced. A suffix S indicates the liquid that was gelled was solely a monohydric alkanol.

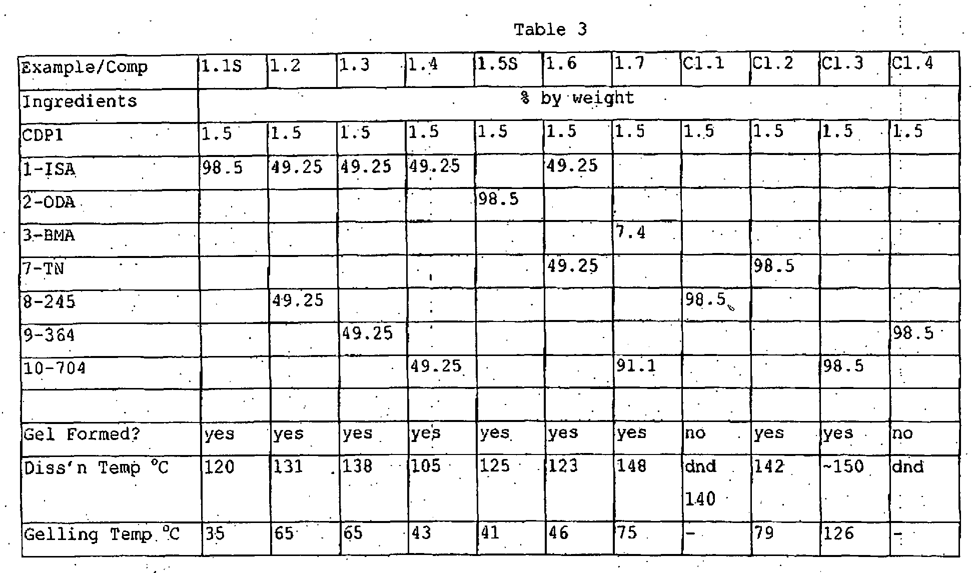

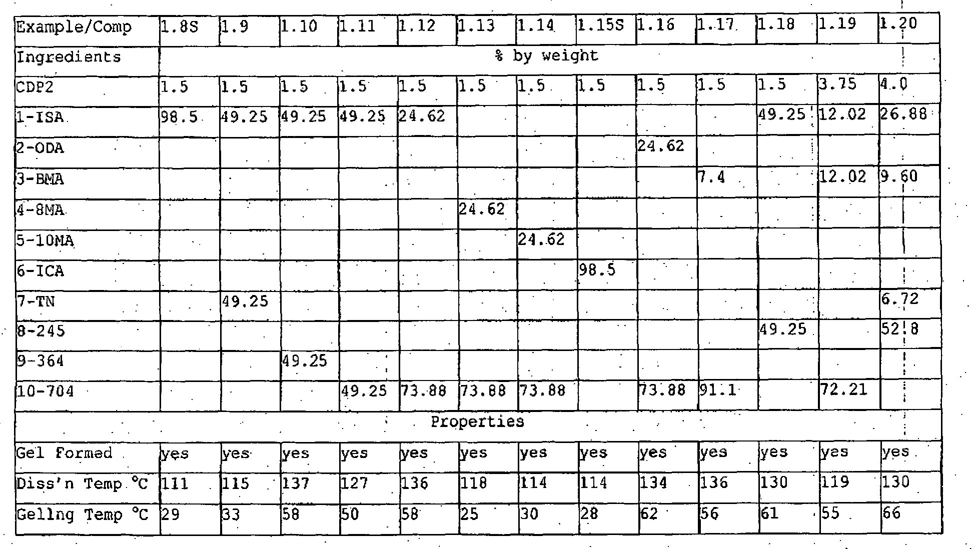

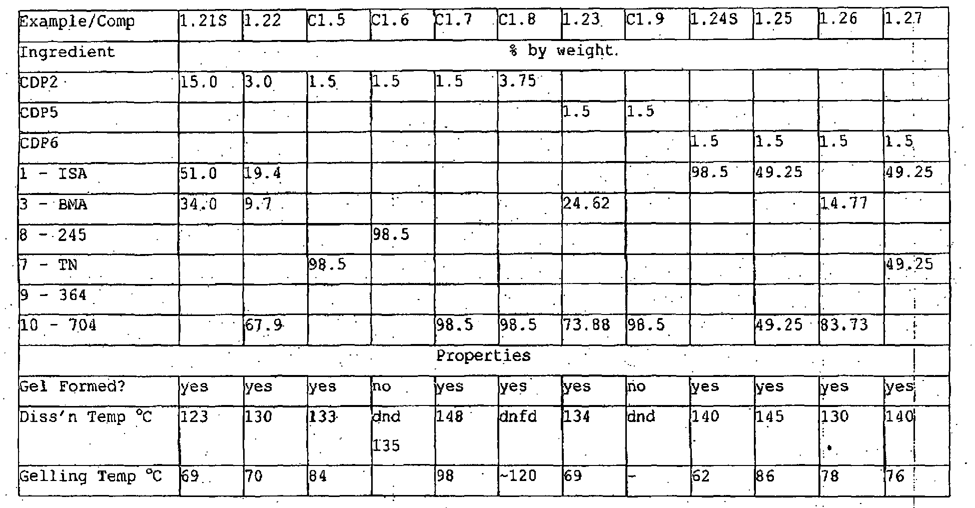

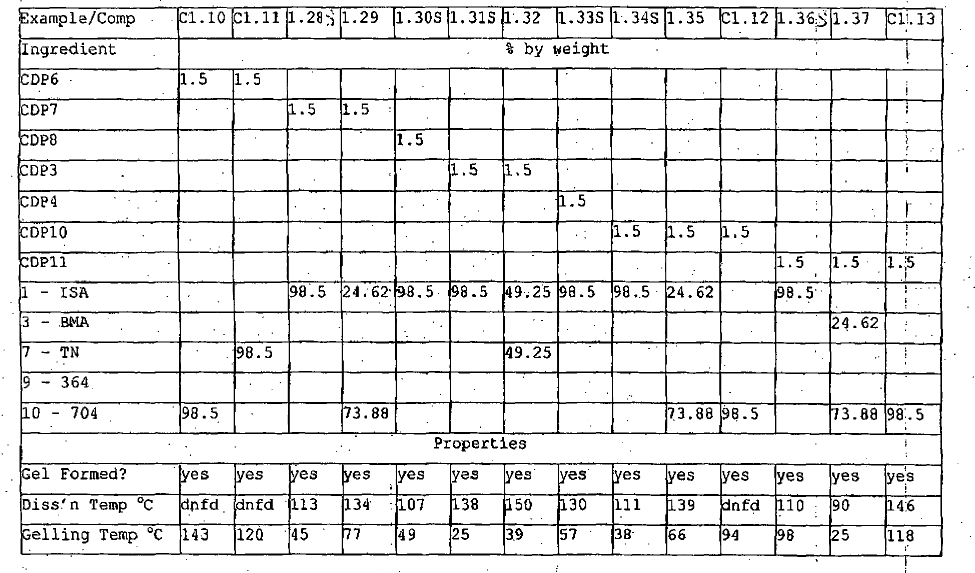

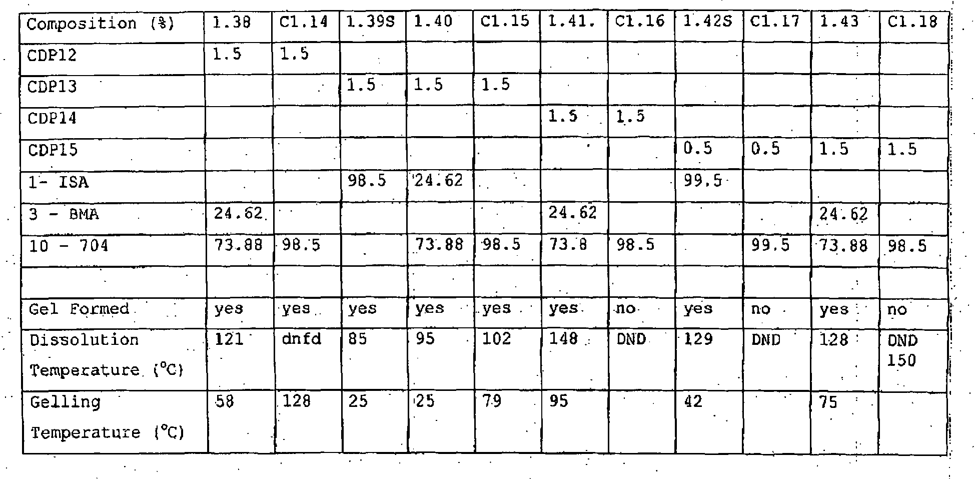

In Table 3 above, dnd indicates that the structurant did not dissolve, and dnfd that it did not fully dissolve, in each case at 150°C unless otherwise indicated.

From Table 3, it can be seen that the employment of the specified monohydric alcohols, viz materials (1) to (6), enabled the resultant composition to gel at a lower temperature. Thus, for example, a comparison of Ex 1.2 with C1.1 shows that the CDP dissolved in the invention mixture at 131°C, but had not dissolved at 140°C in solely the volatile silicone. Similar improvements can be seen by comparing Ex 1.3 or 1.4 with C1.4 and C1.3 respectively. Of course, where the structurant had not dissolved, it could not subsequently form a distributed network through the carrier liquid and hence was not able to form a gel. Where it did dissolve, though at a higher temperature as in C1.3, the composition gelled at a much higher temperature of significantly over 100°C compared with the gelling temperature of the directly comparable invention composition, Ex 1.3. Ex 1.21 shows the formation of a concentrated solution/gel that can subsequently be diluted with other cosmetic oils during stick preparation.

### Examples 2 to 5 - Cosmetic Stick Formulations

A number of cosmetic stick compositions were prepared, containing the ingredients specified in Tables 4 to 10 below. Their properties were measured by the methods described hereinafter and at the times indicated in the summaries.

### Example 2 - Opaque Suspension Sticks

In Example 2, opaque sticks were made by dissolving the specified cyclo dipeptide structurant in isostearyl alcohol whilst with heated and stirring using an overhead paddle stirrer until complete dissolution had occurred. In formulations additionally containing GP1, the latter was dissolved into solution of the cyclo dipeptide structurant at a temperature of about 5 to 10°C lower. The remaining carrier oils were heated to approximately 50°C and stirred using a stirrer bar and the desired solid antiperspirant active was introduced slowly and with gentle stirring into them. When all the active had been added, the mixture was sheared using a Silverson mixer at 7000rpm for 5 minutes to ensure the active was fully dispersed. The active/oil mixture was then heated in an oven to 85°C and mixed into the structurant solution which had been allowed to cool to 90°C. The temperature of the stirred mixture was kept at 85°C until it was poured into conventional commercial 50g stick barrels and allowed to cool except for formulations containing GP1 which were poured at approximately 75°C.

The formulations and properties of the sticks are summarised in Table 4 below.

**Table 4**

| **Example No** | **2.1** | **2.2** | **2.3** | **2.4** | **2.5** |
|---|---|---|---|---|---|
| Ingredient | % by weight | | | | |
| CDP2 | 2.5 | 2.5 | 1.5 | 1 | |
| CDP3 | | | | | 1.5 |
| 1.ISA | 35.75 | 35.75 | 30 | 28.2 | 30 |
| 7 TN | 35.75 | | | 20.9 | |
| 8.245 | | | | 20.9 | |
| 10.704 | | 35.75 | 40 | | 40 |
| 11.GP1 | | | 2.5 | 3.0 | 2.5 |
| 13.R908 | 26.0 | 26.0 | 26.0 | 26.0 | 26.0 |

| Properties | | | | | |
|---|---|---|---|---|---|
| Hardness (mm) | 16.5 | 15.2 | 13.8 | 18.6 | 14.3 |
| pay.off (g)at to on WetorDry | 0.35 | 0.25 | 0.31 | 0.27 | 0.3 |
| whiteness t=24hr on WetorDry | 13 | 16 | 14 | 27 | 20 |
| pay.off (g)at to on wool | 0.99 | 0.63 | 0.82 | 0.63 | 1.08 |
| whiteness t=24hr on wool | 17 | 17 | 16 | 15 | 20 |

From Table 4, it can be seen that sticks of acceptable firmness can be obtained using the invention structurants at comparatively low concentrations of the structurant and also in the presence of an additional structurant, also at a low concentration.

### Example 3 - Transparent Suspension Sticks

The sticks in this Example were made using the process of Example 3 together with a preparatory step. In the preparatory step, the RI of the antiperspirant active was first measured using a standard procedure (Becke line test). The proportions of each of the carrier oils were then determined (through calculation and measurement) such that their weight averaged refractive index was closely matched to that of the active. In Example 3.8, the CDP structurant was fully dissolved in the mixture of monohydric alcohols before being mixed with the remainder of the carrier oils and subsequently with the antiperspirant active. The formulations are summarised in Table 5 below.

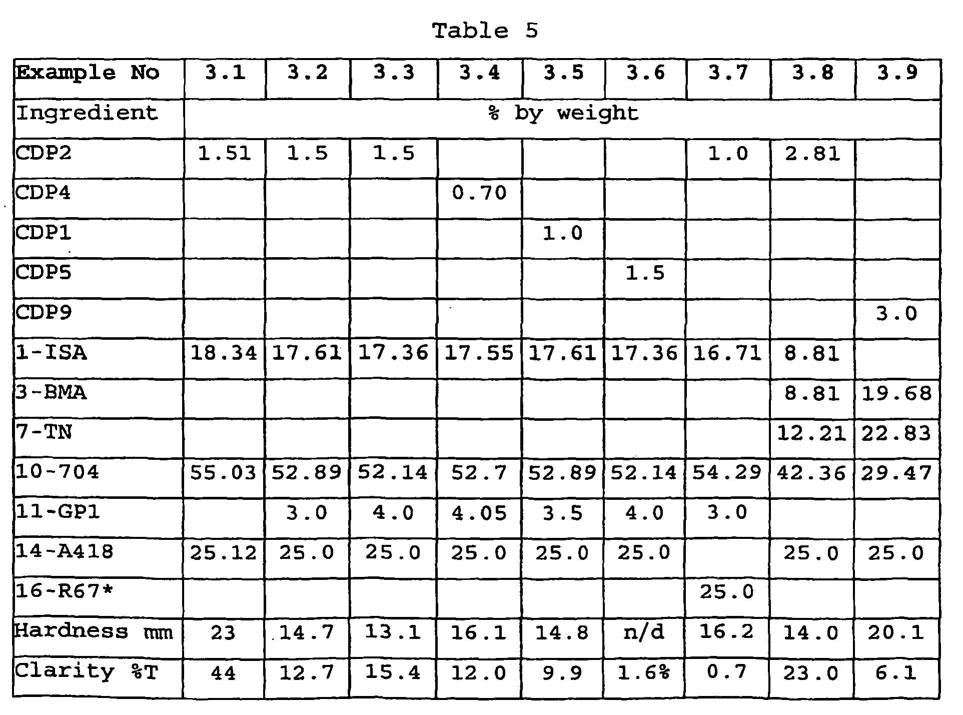

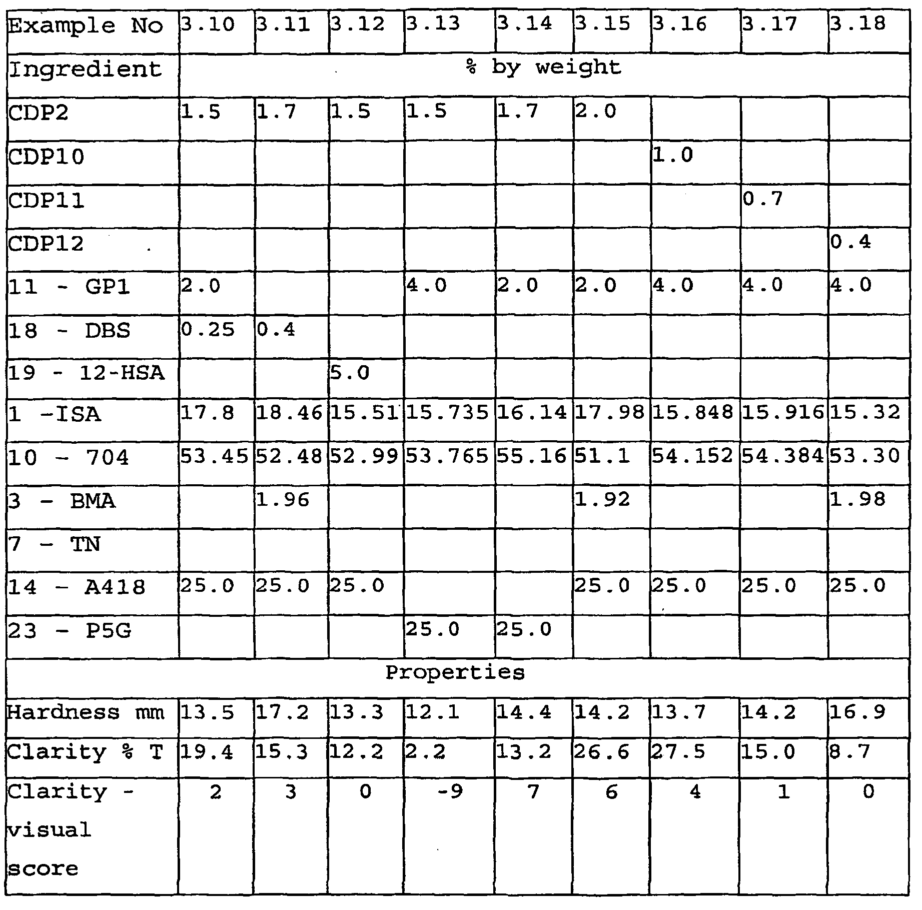

From Table 8, it can be seen that clear cosmetic sticks are obtainable using various combinations of oils as continuous carrier phase together with the CDP structurants, either alone or with a co-structurant.

### Example 4 - Opaque Emulsion Sticks

In a first step in making opaque emulsion sticks according the present invention, a solution of the selected invention structurant, and if present GP1, in ISA was made by the same method as in the process for making suspension sticks (Example 3). The remaining water immiscible carrier oils together with an emulsifier, Abil EM 90, were heated to 85°C in an oil bath whilst being shear mixed at 2500 rpm. The solution of antiperspirant active was heated to 80°C and introduced gradually into the oil/emulsifier mixture, and the resultant mixture was kept constant by heating at 85°C and sheared at 7500 rpm for 5 minutes. The emulsion was the mixed into the solution of the structurant solution which had been allowed to cool to - 90°C. The resultant mixture was stirred briefly to achieve complete mixing, poured into commercial 50g stick barrels at approximately 80°C and allowed to cool. The formulations and properties of the sticks are summarised in Table 9 below.

**Table 6**

| **Example No** | **4.1** | **4.2** |
|---|---|---|
| Ingredient | % by weight | |
| CDP1 | 1.5 | |
| CDP2 | | 1.5 |
| 1-ISA | 29.0 | 27.0 |
| 7-TN. | 29.0 | 27.0 |
| 11-GP-1 | | 4 |
| 12-EM90 | 0.5 | 0.5 |
| 15-Z50 | 40.0 | 40.0 |

| Properties | | |
|---|---|---|
| Hardness (mm) | 27.8 | 17.1 |
| pay-off (g)at to on wool | 0.66 | 0.80 |
| whiteness t=24hr on wool | 17 | 18 |

From Table 6, it can be seen that the use of the monohydric alcohol in conjunction with the other water-immiscible oils enables the emulsion formulations to be made quite easily.

### Example 5 - Clear Emulsion Stick

In this Example, the general method of making emulsion sticks described in Example 5 was followed, preceded by a preparatory step for refractive index matching in order to obtain a translucent emulsion stick.

In the preparatory step, the refractive indices of the ingredients of the organic and aqueous phases in the emulsion were obtained or measured, and proportions of those ingredients estimated, based on calculation and measurement, such that the two phases had roughly matched refractive indices. The two phases containing the estimated proportions of ingredients were prepared, their refractive indices measured and the proportions of the carrier oils in the continuous (water-immiscible) phase were adjusted to the extent necessary to more closely match the RI of the disperse aqueous phase. In Example 5.2, the CDP structurant was fully dissolved in the mixture of monohydric alcohols before being mixed with the remainder of the carrier oils and subsequently with the antiperspirant active.

The Versamid polymer when employed was dissolved simultaneously with the cyclic dipeptide structurant. Any silica was incorporated in suspension in a fraction of the water-immiscible oil(s) and any antiperspirant active supplied as a solid was first dissolved in the specified weight of water.

The formulation and its properties are summarised in Table 7 below, in which nd indicates that a particular test was not carried out.

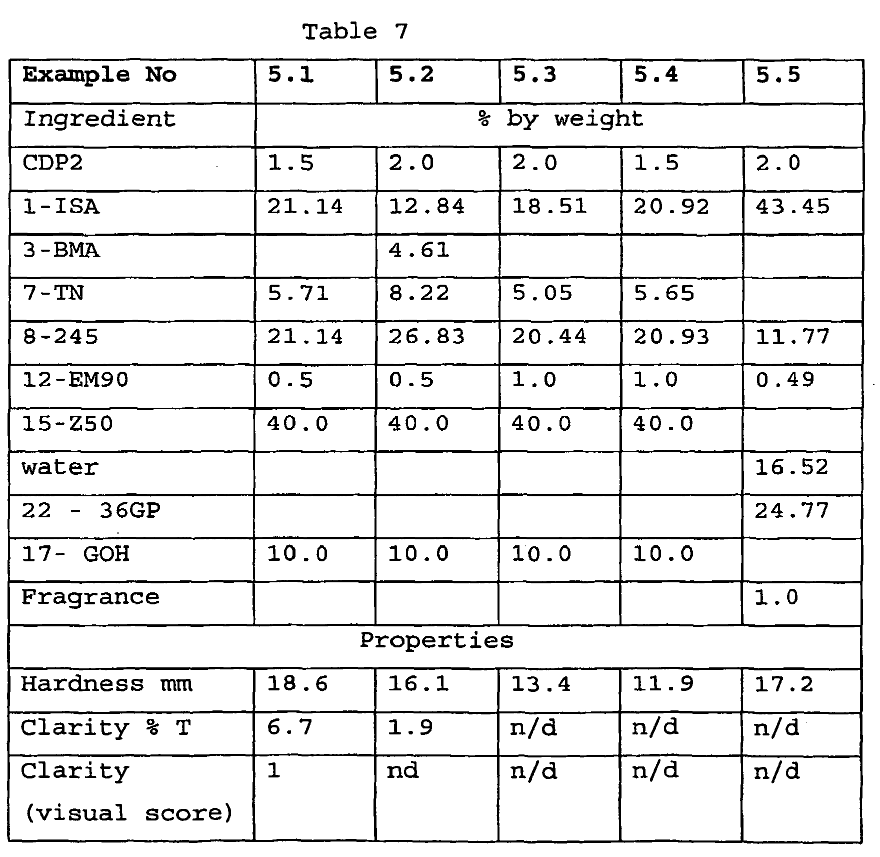

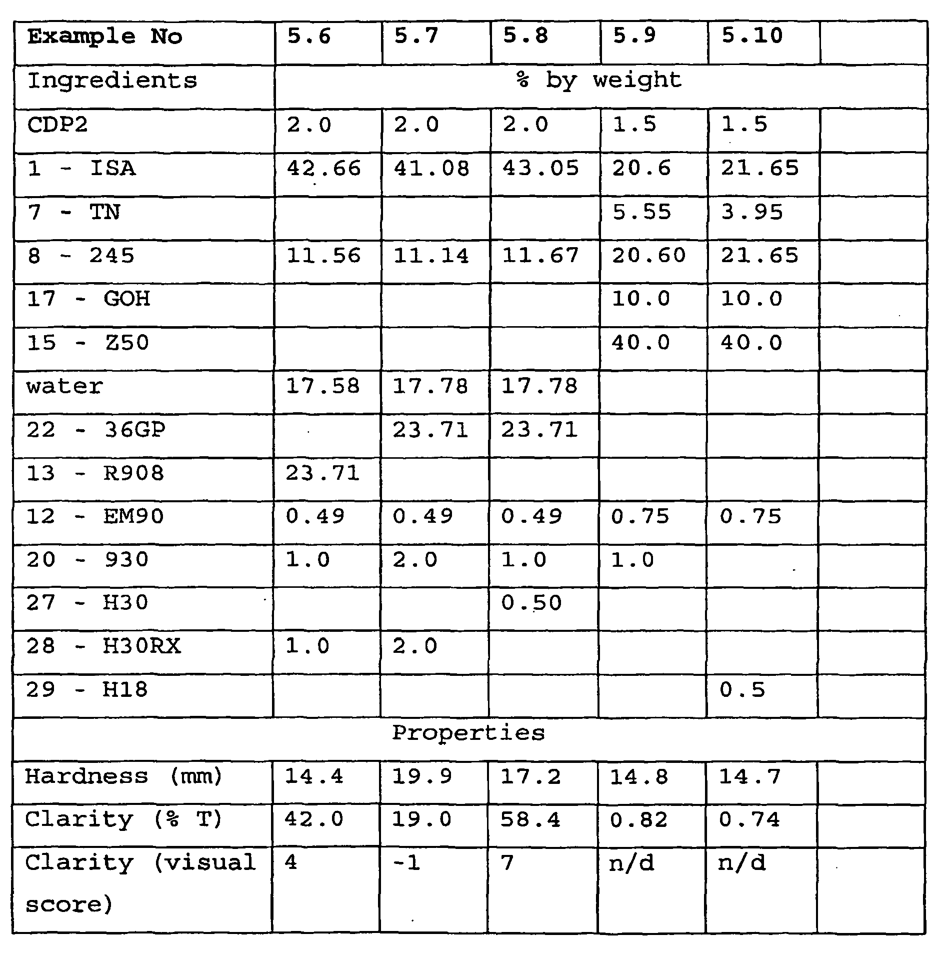

From Table 7 it can be seen not only that clear emulsion sticks can be made that include a faction of a water-miscible liquid, glycerol, but also that it can be made easily employing prior dissolution of the structurant in the monohydric alcohols.

### Test methods

### i) Purity of CDP

The purity of CDP materials A1 to A9 was measured by reverse phase HPLC with ultraviolet (UV)detection.
A mobile phase was made comprising 300ml aliquot of deionised water, to which was added a 700ml aliquot of HPLC grade acetonitrile and 1.0ml of trifluoroacetic acid (Aldrich spectrophotometric grade, TFA) and mixed thoroughly.
0.001g of CDP sample was weighed into a 2 ml HPLC vial and made up to volume with the mobile phase. The sample was then analysed in a Hewlett Packard HPLC
analyser equipped with a Hypersil ODS 5µm C₁₈, 250 x 4.6mm @
Room Temp column, a Hewlett-Packard 1050 Series Autosampler and Hewlett-Packard 1050 UV Diode Array @ 210nm Detector.
The analysis was carried under the following conditions

| | |
|---|---|
| Isocratic/gradient | : Isocratic |
| Flow rate : | 1.2ml/minute |
| Run time : | 5 minutes |
| Temperature : | Ambient |
| Injection volume : | 20µl |

### ii) Dissolution Temperature

The dissolution temperature of the CDP was determined by forming a mixture of the particulate CDP and the selected carrier liquid at ambient temperature keeping the particulates in suspension with a mixer bar and raising the temperature of the mixture at a rate that was initially faster and later of approximately 2°C per minute as the dissolution temperature was approached more closely. The dissolution temperature was assessed as the temperature at which particulates were no longer visible.

### iii) Gelling Temperature

The gelling temperature of a gelled oil phase was determined by first preparing a solution of the CDP in the selected oil(s) in glass tubes, having a diameter of 20mm and equipped with a glass thermometer resting on the bottom of the tube, in accordance with the description for Example 1 herein, and thereafter permitting the resultant solution in the tubes to cool naturally under quiescent conditions, ie without any cooling air being blown over the tubes and without the solution being stirred. External laboratory air temperature was about 23°C. Periodically, the thermometer was lifted by a few mm and if liquid had not flowed to fill the void under gravity, was carefully replaced on the tube bottom. The solution was considered to have formed a gel when it did not flow underneath the thermometer.

### Stick Characterisation - Measurement of Properties

### iv) Stick hardness - Penetrometer

The hardness and rigidity of a composition which is a firm solid can be determined by penetrometry. If the composition is a softer solid, this will be observed as a substantial lack of any resistance to the penetrometer probe. A suitable procedure is to utilises a lab plant PNT penetrometer equipped with a Seta wax needle (weight 2.5 grams) which has a cone angle at the point of the needle specified to be 9°10' ± 15'. A sample of the composition with a flat upper surface is used. The needle is lowered onto the surface of the composition and then a penetration hardness measurement is conducted by allowing the needle with its holder to drop under a total weight, (i.e. the combined weight of needle and holder) of 50 grams for a period of five seconds after which the depth of penetration is noted. Desirably the test is carried out at a number of points on each sample and the results are averaged. Utilising a test of this nature, an appropriate hardness for use in an openended dispensing container is a penetration of less than 30 mm in this test, for example in a range from 2 to 30 mm. Preferably the penetration is in a range from 5mm to 20 mm.

In a specific protocol for this test measurements on a stick were performed in the stick barrel. The stick was wound up to project from the open end of the barrel, and then cut off to leave a flat, uniform surface. The needle was carefully lowered to the stick surface, and then a penetration hardness measurement was conducted. This process was carried out at six different points on the stick surface. The hardness reading quoted is the average value of the 6 measurements.

### v) Deposition by firm sticks (pay-off)

Another property of a composition is the amount of it which is delivered onto a surface when the composition is drawn across that surface (representing the application of a stick product to human skin), sometimes called the pay-off. To carry out this test of deposition when the composition is a firm stick, able to sustain its own shape, a sample of the composition with standardised shape and size is fitted to apparatus which draws the sample across a test surface under standardised conditions. The amount transferred to the surface is determined as an increase in the weight of the substrate to which it is applied. If desired the colour, opacity or clarity of the deposit may subsequently be determined. A specific procedure for such tests of deposition and whiteness applicable to a firm solid stick used apparatus to apply a deposit from a stick onto a substrate under standardised conditions and then measures the mean level of white deposits using image analysis.

The substrates used were:
a: 12 x 28cm strip of grey abrasive paper (3M^{™} P800 WetorDry^{™} Carborundum paper)
b: 12 x 28cm strip of black Worsted wool fabric.
The substrates were weighed before use. The sticks were previously unused and with domed top surface unaltered.

The apparatus comprised a flat base to which a flat substrate was attached by a clip at each end. A pillar having a mounting to receive a standard size stick barrel was mounted on an arm that was moveable horizontally across the substrate by means of a pneumatic piston.

Each stick was kept at ambient laboratory temperature overnight before the measurement was made. The stick was advanced to project a measured amount from the barrel. The barrel was then placed in the apparatus and a spring was positioned to biassed the stick against the substrate with a standardised force. The apparatus was operated to pass the stick laterally across the substrate eight times. The substrate was carefully removed from the rig and reweighed. The whiteness of the deposit could subsequently be measured as described at (v) below.

### vi) Whiteness of Deposit

The deposits from the at test (ii) above, were assessed for their whiteness shortly after application (ie within 30 minutes) or after an interval of 24 hours approximately.

This was done using a Sony XC77 monochrome video camera with a Cosmicar 16mm focal length lens positioned vertically above a black table illuminated from a high angle using fluorescent tubes to remove shadowing. The apparatus was initially calibrated using a reference white card, after the fluorescent tubes had been turned on for long enough to give a steady light output. A cloth or Carborundum paper with a deposit thereon from the previous test was placed on the table and the camera was used to capture an image. An area of the image of the deposit was selected and analysed using a Kontron IBAS^{™} image analyser. This notionally divided the image into a large array of pixels and measured the grey level of each pixel on a scale of 0 (black) to 255 (white). The average of the grey intensity was calculated. This was a measure of the whiteness of the deposit, with higher numbers indicating a whiter deposit. It was assumed that low numbers show a clear deposit allowing the substrate colour to be seen.

### vii) Clarity of formulation - Light transmission

The translucency of a composition may be measured by placing a sample of standardised thickness in the light path of a spectrophotometer and measuring transmittance, as a percentage of light transmitted in the absence of the gel. This test was carried out using a dual-beam Perkin Elmer Lambda 40 spectrophotometer. The sample of composition was poured hot into a 4.5 ml cuvette made of poly(methylmethacrylate) (PMMA) and allowed to cool to an ambient temperature of 20-25°C. Such a cuvette gives a 1 cm thickness of composition. Measurement was carried out at 580 nm, with an identical but empty cuvette in the reference beam of the spectrophotometer, after the sample in the cuvette had been held for 24 hours. A transmittance measured at any temperature in the range from 20-25°C is usually adequately accurate, but measurement is made at 22°C if more precision is required.

### viii) Clarity of Formulation - Visual assessment score

A gel contained within a 1cm thick cuvette was placed directly on to a sheet of white paper on which 21 sets of figures where printed in black. The size and thickness of the figures varied systematically and were numbered from -12 (the largest, thickest set) through 0 to 8 (the smallest thinnest set) The score given to each gel was the highest numbered set which could be read clearly through the gel, the higher the number, the higher the clarity.

## Claims

1. A cosmetic composition comprising:-
(i) a cosmetic active material
(ii) a continuous phase which comprises at least one water-immiscible liquid carrier oil
(iii) a structurant for the continuous phase
**Characterised in that** the structurant comprises a cyclodipeptide having the general formula 1 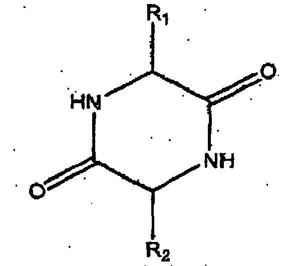 in which at least one of R₁ and R₂ which may be the same or different represents an aliphatic group that is optionally substituted by an aromatic or cycloaliphatic group and the other may alternatively represent hydrogen,
the composition contains a water-immiscible aliphatic alcohol which is liquid at 20°C and boils at above 100°C and the continuous phase comprises
at least 5% by weight thereof of a monohydric alcohol having a melting point of below 30°C and a boiling point of greater than 100°C, and
a water-immiscible carrier cosmetic oil comprising a silicone oil, and/or non-silicone hydrophobic organic liquid selected from hydrocarbons, hydrophobic aliphatic esters, aromatic esters and hydrophobic ethers.

2. A composition according to claim 1 in which the monohydric alcohol has a melting point of below 20°C and a boiling point of greater than 120°C.

3. A composition according to claim 2 in which the monohydric alcohol is selected from linear or branched aliphatic alcohols or benzyl alcohol that satisfy such melting and boiling pint criteria.

4. A composition according to claim 3 in which the monohydric alcohol is iso-stearyl alcohol and/or benzyl alcohol.

5. A composition according to any preceding claim in which the continuous phase comprises from 20 to 80% of the monohydric alcohol.

6. A composition according to any of claims 1 to 4 in which the composition comprises from 15 to 55% of the aliphatic alcohol.

7. A composition according to claim 5 in which the composition comprises from 15 to 30% of the aliphatics alcohol.

8. A composition according to any preceding claim in which one of R₁ and R₂ represent an aliphatic ester group of formula -(CH₂)ₙ-CO₂-R₃ in which n is an integer of at least 1 and R₃ represents an alkyl, cycloalkyl or aryl group.

9. A composition according to any preceding claim in which one of R₁ and R₂ represents a phenyl polymethylene group.

10. A composition according to claim 9 in which R₁ represents -CH₂-Ph.

11. A composition according to claim 9 or 10 in which R₂ represents an aliphatic ester of formula -CH₂-CO₂-R₃ in which R₃ represents a carbocyclic or heterocyclic group.

12. A composition according to claim 11 in which R₃ represents a single ring, optionally bridged.

13. A composition according to claim 11 or 12 in which R₃ comprises a carbocyclic group that is substituted by at least one alkyl substituent.

14. A composition according to claim 13 in which the alkyl substituent is methyl or isopropyl.

15. A composition according to claim 12 in which the ring in R₃ is a cyclohexane or benzene ring substituted by a methyl and an isopropyl group that are para to each other.

16. A composition according to claim 11 in which R₃ is derivable from thymol, isopinocamphenol or a 3,5-dialkyl cyclohexanol.

17. A composition according to claim 16 which is derivable from thymol.

18. A composition according to claim 11 in which the 3,5-dialkyl cyclohexanol is 3,5-diethyl cyclohexanol.

19. A composition according to any preceding claim in which the cyclodipeptide is present at a concentration of from 0.1 to 15% by weight of the composition.

20. A composition according to claim 19 in which the cyclodipeptide is present at a concentration of from 0.3 to 10% by weight of the composition.

21. A composition according to claim 19 in which the cyclodipeptide is present at a concentration of from 0.5 to 5% by weight of the composition.

22. A composition according to claims 19 in which the cyclodipeptide is present at a concentration of from 0.4 to 8% by weight of the continuous phase.

23. A composition according to any one of the preceding claims **characterised in that** the water-immiscible carrier oil comprises a volatile silicone oil.

24. A composition according to claim 23 **characterised in that** the volatile silicone oil comprises from 20 to 100% of the water-immiscible carrier liquid.

25. A composition according to any one of the preceding claims wherein the water-immiscible carrier liquid contains the silicone oil in an amount which is at least 10% by weight on the composition.

26. A composition according to any one of the preceding claims which contains not more than 3% by weight of any fatty alcohol which is solid at 20°C.

27. A composition according to any preceding claim in which the cycle dipeptide is employed in conjunction with a further structurant comprising an N-acyl amino acid derivative.

28. A composition according to claim 27 in which the further structurant is N-lauroyl glutamic acid dibutylamide.

29. A composition according to any one of claims 1 to 26 in which 12-hydroxystearic acid is employed as a Further structurant.

30. A composition according to any one of claims 1 to 25 in which a polyamide is employed as a further structurant.

31. A composition according to anyone of claim 27, 28, 29 or 30 in which the further structurant is employed in a weight ratio to the cyclo dipeptide of from 1:10 to 10:1.

32. A composition according to any one of claims 1 to 26 in which a further structurant comprising a dibenzylidene alditol is employed.

33. A composition according to claim 31 in which dibenzylidene sorbitol is employed at a weight ratio to the cyclodipeptide structurant of from 1:3 to 1:10.

34. A composition according to any preceding claim in which the composition comprises a suspension of the cosmetic active in the structured hydrophobic carrier liquid.

35. A composition according to claim 34 in which the carrier liquid and the suspended cosmetic active have matched refractive indices and has a light transmission of at least 1%.

36. A composition according to any one of claims 1 to 33 wherein the composition is an emulsion with the cosmetic active in solution in a hydrophilic, preferably water-miscible, disperse phase.

37. A composition according to claim 36 wherein the disperse phase contains a diol or polyol.

38. A composition according to claim 37 wherein the disperse phase contains glycerol or 1,2-propane diol.

39. A composition according to any of claims 36 to 38 in which the composition contains from 0.1% to 10% by weight of a nonionic emulsifier.

40. A composition according to claim 39 in which the emulsifier is an alkyl dimethicone copolyol.

41. A composition according to any of claims 36 to 40 in which the refractive indices of the disperse and continuous phases of the emulsion are matched.

42. A cosmetic composition according to any preceding claim in which the cosmetic active is an antiperspirant or deodorant active.

43. A composition according to claim 42 in which the antiperspirant active comprises an aluminium and/or zirconium halohydrate, an activated aluminium and/or zirconium halohydrate, or an aluminium and/or zirconium complex or an activated aluminium and/or zirconium complex.

44. A composition according to claim 42 in which the complex contains both aluminium and zirconium.

45. A composition according to any one of the preceding claims which contains from 5 to 40% by weight of the antiperspirant active.

46. An cosmetic product comprising a dispensing container having an aperture for delivery of a stick, means for urging the contents of the container to the said aperture or apertures, and a composition according to any one of the preceding claims accommodated within the container.

47. A product according to claim 46 wherein the composition is a firm gel such that a penetrometer needle with a cone angle of 9 degrees 10 minutes, drops into the gel for no more than 30mm when allowed to drop under a total weight of 50 grams for 5 seconds.

48. A process for the production of a composition according to any one of claims 1 to 47 comprising the steps of:-
a) forming a mixture containing a liquid carrier, a structurant dissolved therein, and a solid or a disperse liquid phase comprising cosmetic active in particulate or dissolved form at a temperature of at least 40°C and is above the setting temperature of the mixture;
b) introducing the mixture into a mould which preferably is a dispensing container, and
c) cooling or permitting the mixture to cool to ambient temperature,
**characterised in that**
the structurant is a cyclo dipeptide that satisfies the general formula 2 : - 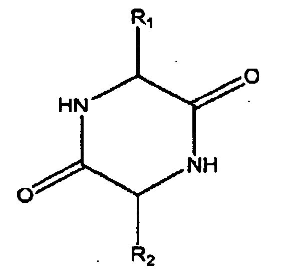 in which at least one of R₁ and R₂ which may be the same or different represents an aliphatic group that is optionally substituted by an aromatic or cycloaliphatic group and the other may alternatively represent hydrogen,
and the carrier comprises
at least 5% by weight thereof of a monohydric alcohol having a melting point of below 30°C and a boiling point of greater than 100°C,
and a water-immiscible carrier cosmetic oil comprising a silicone oil, and/or non-silicone hydrophobic organic liquid selected from hydrocarbons, hydrophobic aliphatic or aromatic esters and/or hydrophobic ethers and the composition contains a water-immiscible aliphatic alcohol which is liquid at 20°C and boils at above 100°C.

49. A process according to claim 48 in which the carrier comprises from 20 to 80% by weight monohydric alcohol.

50. A process according to any of claims 48 or 49 in which the water-immiscible liquid carrier comprises an aromatic ester.

51. A process according to claim 48 in which the cyclodipeptide structurant is dissolved in the monohydric alcohol and up to half of the water-immiscible cosmetic oil prior to being mixed with the or the residual fraction of the water-immiscible cosmetic oil.

52. A process according to any of claims 48 to 51, which includes a step of pouring the mixture at elevated temperature into a dispensing container and allowing it to cool therein so as to produced a product according to claim 39 or 40.

53. A non-therapeutic cosmetic method for preventing or reducing perspiration on human skin comprising topically applying to the skin a composition according to any one of claims 1 to 46.

## Patentansprüche

1. Kosmetische Zusammensetzung, umfassend:
(i) einen kosmetischen Wirkstoff;
(ii) eine kontinuierliche Phase, die wenigstens ein mit Wasser nicht mischbares flüssiges Trägeröl umfasst;
(iii) ein Strukturierungsmittel für die kontinuierliche Phase,
**dadurch gekennzeichnet, dass** das Strukturierungsmittel ein Cyclodipeptid umfasst, das die allgemeine Formel (1) hat 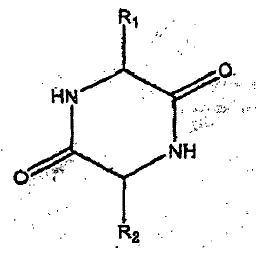 worin wenigstens eines von R₁ und R₂, die gleich oder unterschiedlich sein können, eine aliphatische Gruppe darstellt, die gegebenenfalls mit einer aromatischen oder cycloaliphatischen Gruppe substituiert ist, und das andere alternativ Wasserstoff darstellen kann;
die Zusammensetzung einen mit Wasser nicht mischbaren aliphatischen Alkohol enthält, der bei 20 °C flüssig ist und über 100 °C siedet,
und die kontinuierliche Phase umfasst:
wenigstens 5 Gewichts-% davon eines einwertigen Alkohols, der einen Schmelzpunkt von unter 30 °C und einen Siedepunkt von höher als 100 °C hat, und
ein mit Wasser nicht mischbares kosmetisches Trägeröl, umfassend ein Silikonöl und/oder eine hydrophobe organische Nicht-Silikon-Flüssigkeit, ausgewählt aus Kohlenwasserstoffen, hydrophoben aliphatischen Estern, aromatischen Estern und hydrophoben Ethern.

2. Zusammensetzung gemäß Anspruch 1, wobei der einwertige Alkohol einen Schmelzpunkt von unter 20°C und einen Siedepunkt von höher als 120 °C hat.

3. Zusammensetzung gemäß Anspruch 2, wobei der einwertige Alkohol aus linearen oder verzweigten aliphatischen Alkoholen und Benzylalkoholen, die solchen Schmelzpunkt- und Siedepunkt-Kriterien genügen, ausgewählt ist.

4. Zusammensetzung gemäß Anspruch 3, wobei der einwertige Alkohol Isostearylalkohol und/öder Benzylalkohol ist.

5. Zusammensetzung gemäß einem vorangehenden Anspruch, wobei die kontinuierliche Phase 20 bis 80 % des einwertigen Alkohols umfasst.

6. Zusammensetzung gemäß einem der Ansprüche 1 bis 4, wobei die Zusammensetzung 15 bis 55 % des aliphatischen Alkohols umfasst.

7. Zusammensetzung gemäß Anspruch 5, wobei die Zusammensetzung 15 bis 30 % des aliphatischen Alkohols umfasst.

8. Zusammensetzung gemäß einem vorangehenden Anspruch, wobei eines von R₁ und R₂ eine aliphatische Ester-Gruppe der Formel -(CH₂)ₙ-CO₂R₃ darstellt, worin n eine ganze Zahl von wenigstens 1 ist und R₃ eine Alkyl-, Cycloalkyl- oder ArylGruppe darstellt.

9. Zusammensetzung gemäß einem vorangehenden Anspruch, wobei eines von R₁ und R₂ eine Phenylpolymethylen-Gruppe darstellt.

10. Zusammensetzung gemäß Anspruch 9, wobei R₁ -CH₂-Ph darstellt.

11. Zusammensetzung gemäß Anspruch 9 oder 10, wobei R₂ einen aliphatischen Ester der Formel -CH₂-CO₂-R₃ darstellt, worin R₃ eine carbocyclische oder heterocyclische Gruppe darstellt.

12. Zusammensetzung gemäß Anspruch 11, wobei R₃ einen einzelnen Ring, der gegebenenfalls verbrückt ist, darstellt.

13. Zusammensetzung gemäß Anspruch 11 oder 12, wobei R₃ eine carbocyclische Gruppe umfasst, die mit wenigstens einem Alkyl-Substituenten substituiert ist.

14. Zusammensetzung gemäß Anspruch 13, wobei der Alkyl-Substituent Methyl oder Isopropyl ist.

15. Zusammensetzung gemäß Anspruch 12, wobei der Ring in R₃ ein Cyclohexan- oder Benzol-Ring ist, der mit einer Methyl- und einer Isopropyl-Gruppe substituiert ist, die zueinander para sind.

16. Zusammensetzung gemäß Anspruch 11, wobei R₃ von Thymol, Isopinocamphenol oder einem 3,5-Dialkylcyclohexanol ableitbar ist.

17. Zusammensetzung gemäß Anspruch 16, die von Thymol ableitbar ist.

18. Zusammensetzung gemäß Anspruch 11, wobei das 3,5-Dialkylcyclohexanol 3,5-Dimethylcyclohexanol ist.

19. Zusammensetzung gemäß einem vorangehenden Anspruch, wobei das Cyclodipeptid in einer Konzentration von 0,1 bis 15 Gewichts-% der Zusammensetzung vorliegt.

20. Zusammensetzung gemäß Anspruch 19, wobei das Cyclodipeptid in einer Konzentration von 0,3 bis 10 Gewichts-% der Zusammensetzung vorliegt.

21. Zusammensetzung gemäß Anspruch 19, wobei das Cyclodipeptid in einer Konzentration von 0,5 bis 5 Gewichts-% der Zusammensetzung vorliegt.

22. Zusammensetzung gemäß Anspruch 19, wobei das Cyclodipeptid in einer Konzentration von 0,4 bis 8 Gewichts-% der kontinuierlichen Phase vorliegt.

23. Zusammensetzung gemäß einem der vorangehenden Ansprüche, **dadurch gekennzeichnet, dass** das mit Wasser nicht mischbare Trägeröl ein flüchtiges Silikonöl umfasst.

24. Zusammensetzung gemäß Anspruch 23, **dadurch gekennzeichnet, dass** das flüchtige Silikonöl 20 bis 100 % der mit Wasser nicht mischbaren Trägerflüssigkeit umfasst.

25. Zusammensetzung gemäß einem der vorangehenden Ansprüche, wobei die mit Wasser nicht mischbare Trägerflüssigkeit das Silikonöl in einer Menge enthält, die wenigstens 10 Gewichts-% der Zusammensetzung beträgt.

26. Zusammensetzung gemäß einem der vorangehenden Ansprüche, die nicht mehr als 3 Gewichts-% eines Fettalkohols, der bei 20 °C fest ist, enthält.

27. Zusammensetzung gemäß einem vorangehenden Anspruch, wobei das Cyclodipeptid in Verbindung mit einem weiteren Strukturierungsmittel, das ein N-Acylaminosäure-Derivat umfasst, verwendet wird.

28. Zusammensetzung gemäß Anspruch 27, wobei das weitere Strukturierungsmittel N-Lauroylglutaminsäuredibutylamid ist.

29. Zusammensetzung gemäß einem der Ansprüche 1 bis 26, wobei 12-Hydroxystearinsäure als ein weiteres Strukturierungsmittel verwendet wird.

30. Zusammensetzung gemäß einem der Ansprüche 1 bis 25, wobei ein Polyamid als ein weiteres Strukturierungsmittel verwendet wird.

31. Zusammensetzung gemäß einem der Ansprüche 27, 28, 29 oder 30, wobei das weitere Strukturierungsmittel in einem Gewichtsverhältnis zu dem Cyclodipeptid von 1:10 bis 10:1 verwendet wird.

32. Zusammensetzung gemäß einem der Ansprüche 1 bis 26, wobei ein weiteres Strukturierungsmittel, das ein Dibenzylidenalditol umfasst, verwendet wird.

33. Zusammensetzung gemäß Anspruch 31, wobei Dibenzylidensorbitol in einem Gewichtsverhältnis zu dem Cyclodipeptid-Strukturierungsmittel von 1:3 bis 1:10 verwendet wird.

34. Zusammensetzung gemäß einem vorangehenden Anspruch, wobei die Zusammensetzung eine Suspension des kosmetischen Wirkstoffs in der strukturierten hydrophoben Trägerflüssigkeit umfasst.

35. Zusammensetzung gemäß Anspruch 34, wobei die Trägerflüssigkeit und der suspendierte kosmetische Wirkstoff zueinander passende Brechungsindices haben und sie einen Lichttransmissionsgrad von wenigstens 1 % hat.

36. Zusammensetzung gemäß einem der Ansprüche 1 bis 33, wobei die Zusammensetzung eine Emulsion mit dem kosmetischen Wirkstoff in Lösung in einer hydrophilen, vorzugsweise mit Wasser mischbaren, dispersen Phase ist.

37. Zusammensetzung gemäß Anspruch 36, wobei die disperse Phase ein Diol oder Polyol enthält.

38. Zusammensetzung gemäß Anspruch 37, wobei die disperse Phase Glycerin oder 1,2-Propandiol enthält.

39. Zusammensetzung gemäß einem der Ansprüche 36 bis 38, wobei die Zusammensetzung 0,1 % bis 10 Gewichts-% eines nicht-ionischen Emulgators enthält.

40. Zusammensetzung gemäß Anspruch 39, wobei der Emulgator ein Alkyldimethicon-Copolyol ist.

41. Zusammensetzung gemäß einem der Ansprüche 36 bis 40, wobei die Brechungsindices der dispersen und kontinuierlichen Phasen der Emulsion zueinander passen.

42. Kosmetische Zusammensetzung gemäß einem vorangehenden Anspruch, wobei der kosmetische Wirkstoff ein Antiperspirans- oder Desodorans-Wirkstoff ist.

43. Zusammensetzung gemäß Anspruch 42, wobei der Antiperspirans-Wirkstoff ein Aluminium- und/oder Zirkoniumhalogenid-Hydrat, ein aktiviertes Aluminium- und/oder Zirkoniumhalogenid-Hydrat oder einen Aluminium- und/oder Zirkoniumkomplex oder einen aktivierten Aluminium- und/oder Zirkoniumkomplex umfasst.

44. Zusammensetzung gemäß Anspruch 42, wobei der Komplex sowohl Aluminium als auch Zirkonium enthält.

45. Zusammensetzung gemäß einem der vorangehenden Ansprüche, die 5 bis 50 Gewichts-% des Antiperspirans-Wirkstoffs enthält.

46. Kosmetisches Produkt, umfassend einen Spender-Behälter, der eine Öffnung zur Abgabe eines Stifts, Mittel zum Treiben der Inhalte des Behälters zu der Öffnung oder den Öffnungen und eine Zusammensetzung gemäß einem der vorangehenden Ansprüche, die in dem Behälter untergebracht ist.

47. Produkt gemäß Anspruch 46, wobei die Zusammensetzung ein festes Gel ist, so dass eine Penetrometernadel mit einem Konuswinkel von 9 Grad 10 Minuten nicht weiter als 30 mm in das Gel eindringt, wenn sie unter einem Gesamtgewicht von 50 Gramm für 5 Sekunden fallen gelassen wird.

48. Verfahren zur Herstellung einer Zusammensetzung gemäß einem der Ansprüche 1 bis 47, umfassend die Schritte:
a) Bilden eines Gemisches, enthaltend einen flüssigen Träger, ein Strukturierungsmittel darin gelöst und eine feste oder eine disperse flüssige Phase, umfassend kosmetischen Wirkstoff in partikulärer oder gelöster Form, bei einer Temperatur von wenigstens 40 °C und oberhalb der Erstarrungstemperatur des Gemisches;
b) Einführen des Gemisches in eine Form, welche vorzugsweise ein Spender-Behälter bzw. Dosier-Behälter ist, und
c) Kühlen des Gemisches oder Abkühlenlassen des Gemisches auf Umgebungstemperatur,
**dadurch gekennzeichnet, dass**
das Strukturierungsmittel ein Cyclodipeptid ist, das der allgemeinen Formel 1 genügt: 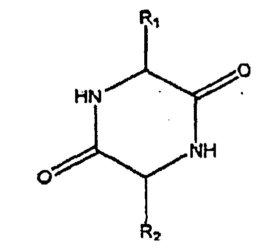 worin wenigstens eines von R₁ und R₂, die gleich oder unterschiedlich sein können, eine aliphatische Gruppe darstellt, die gegebenenfalls mit einer aromatischen oder cycloaliphatischen Gruppe substituiert ist, und das andere alternativ Wasserstoff darstellen kann;
und der Träger umfasst:
wenigstens 5 Gewichts-% davon eines einwertigen Alkohols, der einen Schmelzpunkt von unter 30 °C und einen Siedepunkt von höher als 100 °C hat,
und ein mit Wasser nicht mischbares kosmetisches Trägeröl, umfassend ein Silikonöl und/oder eine hydrophobe organische Nicht-Silikon-Flüssigkeit, ausgewählt aus Kohlenwasserstoffen, hydrophoben aliphatischen oder aromatischen Estern und/oder hydrophoben Ethern,
und die Zusammensetzung einen mit Wasser nicht mischbaren aliphatischen Alkohol enthält, der bei 20 °C flüssig ist und über 100 °C siedet.

49. Verfahren gemäß Anspruch 48, wobei der Träger 20 bis 80 Gewichts-% einwertigen Alkohol umfasst.

50. Verfahren gemäß einem der Ansprüche 48 und 49, wobei der mit Wasser nicht mischbare flüssige Träger einen aromatischen Ester umfasst.

51. Verfahren gemäß Anspruch 48, wobei das Cyclodipeptid-Strukturierungsmittel in dem einwertigen Alkohol und bis zur Hälfte des mit Wasser nicht mischbaren kosmetischen Öls gelöst wird, bevor es mit dem mit Wasser nicht mischbaren kosmetischen Öl oder der Restfraktion des mit Wasser nicht mischbaren kosmetischen Öls vermischt wird.

52. Verfahren gemäß einem der Ansprüche 48 bis 51, welches einen Schritt des Gießens des Gemisches mit erhöhter Temperatur in einen Spender-Behälter bzw. Dosier-Behälter und Abkühlen-Lassen darin unter Herstellung eines Produkts gemäß Anspruch 39 oder 40 umfasst.

53. Nicht-therapeutisches kosmetisches Verfahren zur Verhinderung oder Verringerung der Perspiration an menschlicher Haut, umfassend topisches Auftragen bzw. Anwenden einer Zusammensetzung gemäß einem der Ansprüche 1 bis 46 auf die Haut.

## Revendications

1. Composition cosmétique comprenant :
(i) une matière active cosmétique
(ii) une phase continue qui comprend au moins une huile vecteur liquide immiscible à l'eau
(iii) un structurant pour la phase continue **caractérisée en ce que** le structurant comprend un cyclodipeptide répondant à la formule générale 1 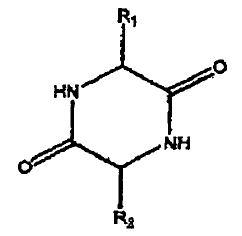
dans laquelle au moins l'un de R₁ et de R₂ qui peuvent être identiques ou différents représente un groupe aliphatique qui est facultativement substitué par un groupe aromatique ou cycloaliphatique et l'autre peut en variante représenter un atome d'hydrogène,
la composition contient un alcool aliphatique immiscible à l'eau qui est liquide à 20°C et bout à plus de 100°C et la phase continue comprend
au moins 5 % en poids d'un alcool monohydrique ayant un point de fusion inférieur à 30°C et un point d'ébullition supérieur à 100°C, et
une huile cosmétique vecteur immiscible à l'eau comprenant une huile de silicone et/ou un liquide organique hydrophobe non-silicone choisi parmi les hydrocarbures, les esters aliphatiques hydrophobes, les esters aromatiques et les éthers hydrophobes.

2. Composition selon la revendication 1, dans laquelle l'alcool monohydrique a un point de fusion inférieur à 20°C et un point d'ébullition supérieur à 120°C.

3. Composition selon la revendication 2, dans laquelle l'alcool monohydrique est choisi parmi les alcools aliphatiques linéaires ou ramifiés ou l'alcool benzylique qui satisfont de tels critères en termes de point de fusion et d'ébullition.

4. Composition selon la revendication 3, dans laquelle l'alcool monohydrique est l'alcool iso-stéarylique et/ou l'alcool benzylique.

5. Composition selon l'une quelconque des revendications précédentes, dans laquelle la phase continue comprend de 20 à 80 % de l'alcool monohydrique.

6. Composition selon l'une quelconque des revendications 1 à 4, dans laquelle la composition comprend de 15 à 55 % de l'alcool aliphatique.

7. Composition selon la revendication 5, dans laquelle la composition comprend de 15 à 30 % de l'alcool aliphatique.

8. Composition selon l'une quelconque des revendications précédentes, dans laquelle l'un de R₁ et R₂ représente un groupe ester aliphatique de formule - -(CH₂)ₙCO₂-R₃ dans laquelle n est un nombre entier d'au moins 1 et R₃ représente un groupe alkyle, cycloalkyle ou aryle.

9. Composition selon l'une quelconque des revendications précédentes, dans laquelle l'un de R₁ et R₂ représente un groupe phényl polyméthylène.

10. Composition selon la revendication 9, dans laquelle R₁ représente -CH₂-Ph.

11. Composition selon la revendication 9 ou 10, dans laquelle R₂ représente un ester aliphatique de formule -CH₂-CO₂-R₃ où R₃ représente un groupe carbocyclique ou hétérocyclique.

12. Composition selon la revendication 11, dans laquelle R₃ représente un cycle unique, facultativement ponté.

13. Composition selon la revendication 11 ou 12, dans laquelle R₃ représente un groupe carbocyclique qui est substitué par au moins un substituant alkyle.

14. Composition selon la revendication 13, dans laquelle le substituant alkyle est le méthyle ou l'isopropyle.

15. Composition selon la revendication 12, dans laquelle le cycle dans R₃ est un cycle cyclohexane ou benzène substitué par un groupe méthyle et un groupe isopropyle qui sont para l'un par rapport à l'autre.

16. Composition selon la revendication 11, dans laquelle R₃ peut être dérivé du thymol, de l'iso-pinocamphénol ou d'un 3,5-dialkyl cyclohexanol.

17. Composition selon la revendication 16, qui peut être dérivée du thymol.

18. Composition selon la revendication 11, dans laquelle le 3,5-dialkyl cyclohexanol est le 3,5-diméthyl cyclohexanol.

19. Composition selon l'une quelconque des revendications précédentes, dans laquelle le cyclodipeptide est présent à une concentration de 0,1 à 15 % en poids de la composition.

20. Composition selon la revendication 19, dans laquelle le cyclodipeptide est présent à une concentration de 0,3 à 10 % en poids de la composition.

21. Composition selon la revendication 19, dans laquelle le cyclodipeptide est présent à une concentration de 0,5 à 5 % en poids de la composition.

22. Composition selon la revendication 19, dans laquelle le cyclodipeptide est présent à une concentration de 0,4 à 8 % en poids de la phase continue.

23. Composition selon l'une quelconque des revendications précédentes, **caractérisée en ce que** l'huile vecteur immiscible à l'eau comprend une huile de silicone volatile.

24. Composition selon la revendication 23, **caractérisée en ce que** l'huile de silicone volatile comprend de 20 à 100 % du liquide vecteur immiscible à l'eau.

25. Composition selon l'une quelconque des revendications précédentes, dans laquelle le liquide vecteur immiscible à l'eau contient l'huile de silicone dans une quantité qui est d'au moins 10 % en poids de la composition.

26. Composition selon l'une quelconque des revendications précédentes, qui contient pas plus de 3 % en poids d'un alcool gras quelconque qui est solide à 20°C.

27. Composition selon l'une quelconque des revendications précédentes, dans laquelle le cyclodipeptide est employé conjointement avec un structurant supplémentaire comprenant un dérivé d'acide N-acylaminé.

28. Composition selon la revendication 27, dans laquelle le structurant supplémentaire est le dibutylamide d'acide N-lauroyl glutamique.

29. Composition selon l'une quelconque des revendications 1 à 26, dans laquelle de l'acide 12-hydroxystéarique est employé comme structurant supplémentaire.

30. Composition selon l'une quelconque des revendications 1 à 25, dans laquelle un poly(amide) est employé comme structurant supplémentaire.

31. Composition selon l'une quelconque des revendications 27, 28, 29 ou 30, dans laquelle le structurant supplémentaire est employé dans un rapport en poids par rapport au cyclodipeptide de 1 : 10 à 10 : 1.

32. Composition selon l'une quelconque des revendications 1 à 26, dans laquelle un structurant supplémentaire comprenant un dibenzylidène alditol est employé.

33. Composition selon la revendication 31, dans laquelle du dibenzylidène sorbitol est employé à un rapport en poids par rapport au structurant cyclodipeptide de 1 : 3 à 1 : 10.

34. Composition selon l'une quelconque des revendications précédentes, dans laquelle la composition comprend une suspension du principe actif cosmétique dans le liquide vecteur hydrophobe structuré.

35. Composition selon la revendication 34, dans laquelle le liquide vecteur et le principe actif cosmétique mis en suspension ont des indices de réfraction concordants et ont une transmission de la lumière d'au moins 1 %.

36. Composition selon l'une quelconque des revendications 1 à 33, dans laquelle la composition est une émulsion avec le principe actif cosmétique en solution dans une phase dispersée hydrophile, de préférence miscible à l'eau.

37. Composition selon la revendication 36, dans laquelle la phase dispersée contient un diol ou polyol.

38. Composition selon la revendication 37, dans laquelle la phase dispersée comprend du glycérol ou du 1,2-propanediol.

39. Composition selon l'une quelconque des revendications 36 à 38, dans laquelle la composition contient de 0,1 % à 10 % en poids d'un émulsionnant non ionique.

40. Composition selon la revendication 39, dans laquelle l'émulsionnant est un alkyl diméthicone copolyol.

41. Composition selon l'une quelconque des revendications 36 à 40, dans laquelle les indices de réfraction des phases dispersée et continue de l'émulsion concordent.

42. Composition cosmétique selon l'une quelconque des revendications précédentes, dans laquelle le principe actif cosmétique est un anti-transpirant ou un principe actif déodorant.

43. Composition selon la revendication 42, dans laquelle le principe actif anti-transpirant comprend un halogénohydrate d'aluminium et/ou de zirconium, un halogénohydrate d'aluminium et/ou de zirconium activé, ou un complexe d'aluminium et/ou de zirconium ou un complexe d'aluminium et/ou de zirconium activé.

44. Composition selon la revendication 42, dans laquelle le complexe contient à la fois de l'aluminium et du zirconium.

45. Composition selon l'une quelconque des revendications précédentes, qui contient de 5 à 40 % en poids du principe actif anti-transpirant.

46. Produit cosmétique comprenant un contenant de distribution ayant une ouverture pour administrer un bâtonnet, un moyen pour pousser les contenus du contenant vers ladite ouverture ou lesdites ouvertures, et une composition selon l'une quelconque des revendications précédentes logée dans le contenant.

47. Produit selon la revendication 46, dans lequel la composition est un gel ferme de telle sorte qu'une aiguille de pénétromètre avec un angle du cône de 9 degrés 10 minutes, chute dans le gel sur pas plus de 30 mm lorsqu'on le laisse chuter sous un poids total de 50 grammes pendant 5 secondes.

48. Procédé de production d'une composition selon l'une quelconque des revendications 1 à 47, comprenant les étapes consistant à :
a) former un mélange contenant un vecteur liquide, un structurant dissous à l'intérieur, et un solide ou une phase liquide dispersée comprenant un principe actif cosmétique sous forme particulaire ou dissoute à une température d'au moins 40°C et qui est au-dessus de la température de durcissement du mélange ;
b) introduire le mélange dans un moule qui est de préférence un contenant de distribution, et
c) refroidir ou laisser le mélange refroidir à température ambiante,
**caractérisé en ce que**
le structurant est un cyclodipeptide qui répond à la formule générale 1 : 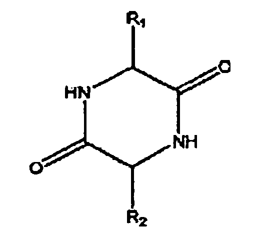 dans laquelle au moins l'un de R₁ et de R₂ qui peuvent être identiques ou différents représente un groupe aliphatique qui est facultativement substitué par un groupe aromatique ou cycloaliphatique et l'autre peut en variante représenter un atome d'hydrogène,
et le vecteur comprend
au moins 5 % en poids d'un alcool monohydrique ayant un point de fusion inférieur à 30°C et un point d'ébullition supérieur à 100°C,
et une huile cosmétique vecteur immiscible à l'eau comprenant une huile de silicone et/ou un liquide organique hydrophobe non-silicone choisi parmi les hydrocarbures, les esters aliphatiques ou aromatiques hydrophobes et/ou les éthers hydrophobes
et la composition contient un alcool aliphatique immiscible à l'eau qui est liquide à 20°C et bout à plus de 100°C.

49. Procédé selon la revendication 48, dans lequel le vecteur comprend de 20 à 80 % en poids d'alcool monohydrique.

50. Procédé selon l'une quelconque des revendications 48 ou 49, dans lequel le vecteur liquide immiscible à l'eau comprend un ester aromatique.

51. Procédé selon la revendication 48, dans lequel le structurant cyclodipeptide est dissous dans l'alcool monohydrique et jusqu'à la moitié de l'huile cosmétique immiscible à l'eau avant d'être mélangé avec la fraction résiduelle de l'huile cosmétique immiscible à l'eau.

52. Procédé selon l'une quelconque des revendications 48 à 51, qui comprend une étape de versement du mélange à température élevée dans un contenant de distribution et consistant à l'y laisser refroidir de façon à produire un produit selon la revendication 39 ou 40.

53. Procédé cosmétique non thérapeutique pour empêcher ou réduire la transpiration sur la peau d'un être humain comprenant l'application par voie topique à la peau d'une composition selon l'une quelconque des revendications 1 à 46.
